# EUROPEAN PATENT SPECIFICATION

(11) **EP 3 927 719 B1**
(45) Date of publication and mention of the grant of the patent: **15.02.2023**
(21) Application number: 20706678.8
(22) Date of filing: 19.02.2020
(51) Int. Cl.: C07J 9/00, C07J 17/00, C07J 51/00

(54) **DIETHYLAMINE SALT OF 3ALPHA-TETRAHYDROPYRANYLOXY-6ALPHA-ETHYL-7ALPHA-HYDROXY-5BETA-CHOLANIC ACID**
DIETHYLAMINSALZ VON 3ALPHA-TETRAHYDROPYRANYLOXY-6ALPHA-ETHYL-7ALPHA-HYDROXY-5BETA-CHOLANSÄURE
SEL DE DIÉTHYLAMINE DE L'ACIDE 3 ALPHA-TÉTRAHYDROPYRANYLOXY-6 ALPHA-ÉTHYL-7 ALPHA-HYDROXY-5 BÊTA-CHOLANIQUE

(30) Priority: 20.02.2019 ES 201930143
(43) Date of publication of application: 29.12.2021
(73) Proprietor: Moehs Ibérica, S.L., 08191 Rubí-Barcelona (ES)
(72) Inventor: BERNABEU MARTÍNEZ, María del Carmen, 08191 Rubí, Barcelona (ES); JIMÉNEZ ALONSO, Oscar, 08191 Rubí, Barcelona (ES); DOBARRO RODRÍGUEZ, Alicia, 08191 Rubí, Barcelona (ES)
(74) Representative: ABG Intellectual Property Law, S.L.
(86) International application number: PCT/EP2020/054308
(87) International publication number: WO 2020/169643

(56) References cited:
- WO-A1-2019/170521
- CN-A- 106 589 039
- CN-A- 107 400 154
- CN-A- 107 955 058

## Description

### Field of the Invention

The present invention relates to the diethylamine salt of 3α-tetrahydropyranyloxy-6α-ethyl-7α-hydroxy-5β-cholanic acid (compound of formula (I)), which is a new intermediate of the obeticholic acid synthesis. The present invention also relates to the use of said salt in obeticholic acid synthesis, as well as to the method of obtaining this new salt.

### Background of the Invention

Obeticholic acid (OCA) or 3α;,7α;-dihydroxy-6α;-ethyl-5β-cholan-24-oic acid or compound of formula (II) in the present invention, is a 6α-ethylated derivative of the bile acid chenodeoxycholic acid (CDCA). The chemical structure of obeticholic acid is shown below.

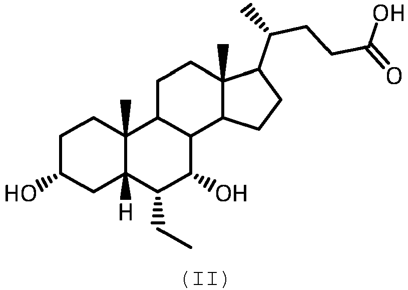

Obeticholic acid is a farnesoid X receptor (FXR) ligand which is used in the treatment of primary biliary cholangitis and is under development for the treatment of other liver diseases.

Obeticholic acid (compound of formula (II)) and its synthesis method are disclosed in WO 02/072598 A1. The synthesis pathway comprises: protection of the hydroxyl group at the C3-position of 3o;-hydroxy-7-keto-5β-cholan-24-oic acid (IX) with a tetrahydropyranyl group to yield 3α-tetrahydropyranyloxy-7-keto-5β-cholan-24-oic acid (XI), alkylation of the carbon at the C6-position and esterification of the carboxylic group with ethyl bromide, and deprotection of the tetrahydropyranyl group to yield ethyl 3α-hydroxy-6α-ethyl-7-keto-5β-cholan-24-oate (XII), reduction of the ketone group at the C7-position to hydroxyl with sodium borohydride to yield ethyl 3α,7α-dihydroxy-6α-ethyl-5β-cholan-24-oate (XIII), and finally deprotection of the ester group to yield obeticholic acid (II).

The problem with this synthesis pathway is, on the one hand, the low yield (3%), and on the other hand, it involves multiple purification steps by column chromatography, which complicates its implementation on an industrial scale.

Zampella et al. [J. Med. Chem., 2012, 55, 84-93] disclose another synthesis pathway for obeticholic acid 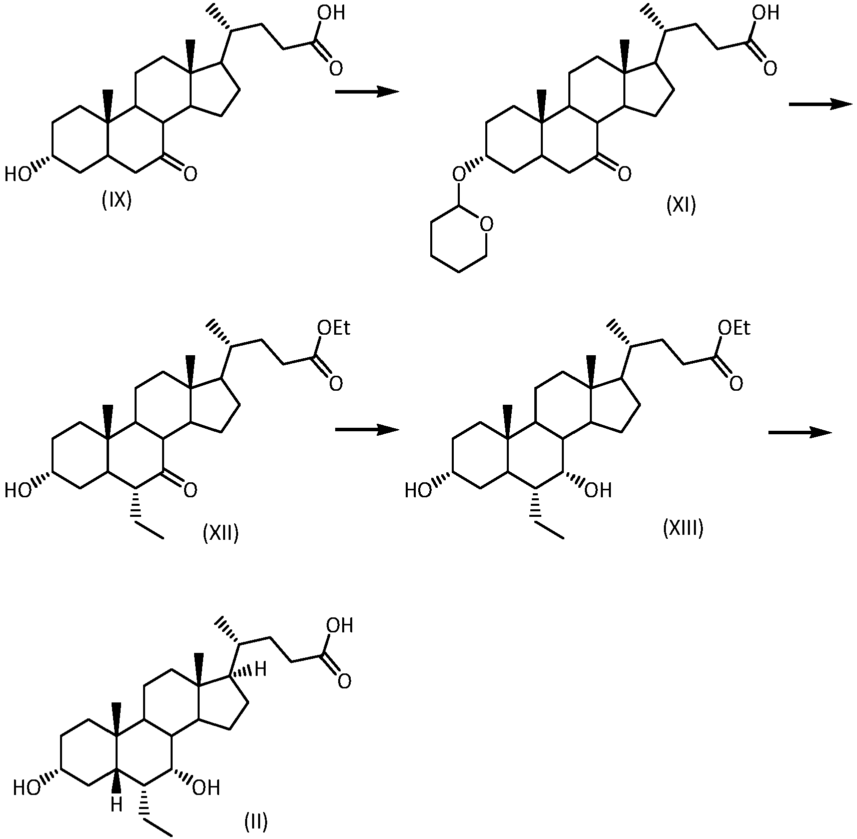 comprising the oxidation of chenodeoxycholic acid (compound of formula (X) or CDCA) with a solution of sodium hypochlorite/NaBr and tetrabutylammonium bromide in a mixture of methanol/acetic acid/water/ethyl acetate as a solvent, followed by benzylation of the carboxylic acid at the C24-position, to yield 7-ketolithocholic acid benzyl ester (XIV). The silyl enol ether (XV) is then generated followed by aldol addition with acetaldehyde in the presence of BF₃·OEt₂ to yield benzyl 3α-hydroxy-6α-ethylindene-7-keto-5β-cholan-24-oate (XVI). Next, the selective reduction of the ketone at the C7-position with NaBH₄/CeCl₃ in a mixture of THF/methanol, and subsequently hydrogenation of the exocyclic double bond, together with the removal of the benzyl protecting group to yield the obeticholic acid (II) are carried out.

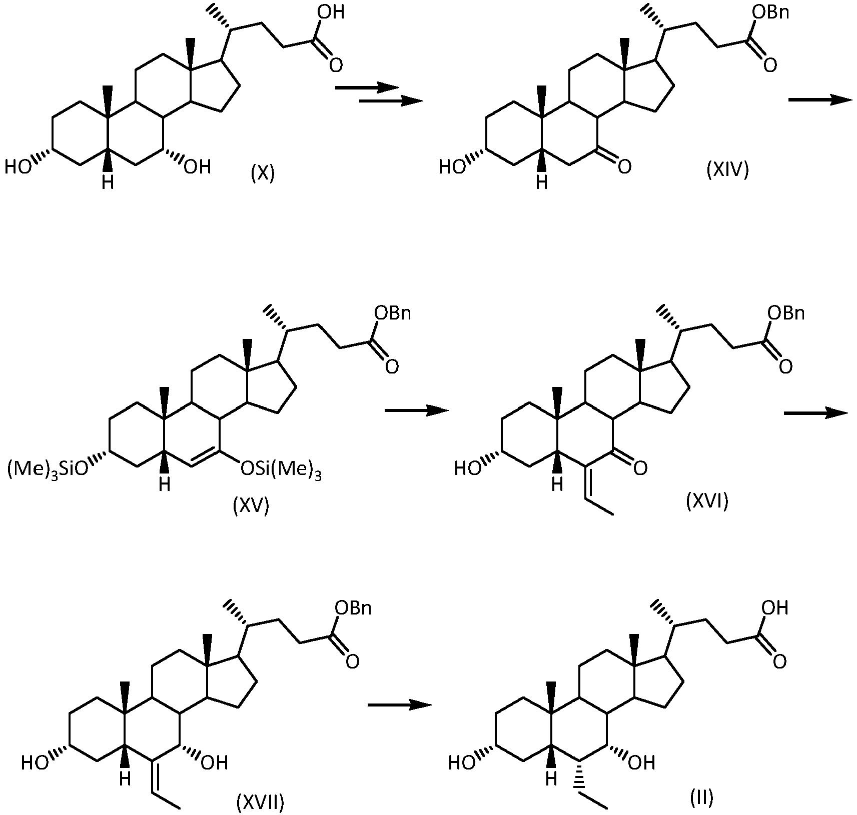

The yield of this synthesis pathway is 32%. Despite having improved the yield, this synthesis pathway still involves several purification steps by column chromatography, so it is not suitable for industrial implementation.

US 8,338,628 B2 discloses a method of obtaining obeticholic acid comprising the steps of oxidizing the hydroxyl at the C7-position of CDCA (compound of formula (X)) to a ketone group with pyridinium chlorochromate yielding compound (IX), protection of the hydroxyl at the C3-position with a tetrahydropyranyl group (rendering compound (XI)), alkylation of the carbon at the C6-position with ethyl iodide and deprotection of the tetrahydropyranyl group (rendering compound (XVIII)), and finally reduction of the ketone group at the C7-position to hydroxyl with sodium borohydride to yield obeticholic acid (II), as shown in the scheme below.

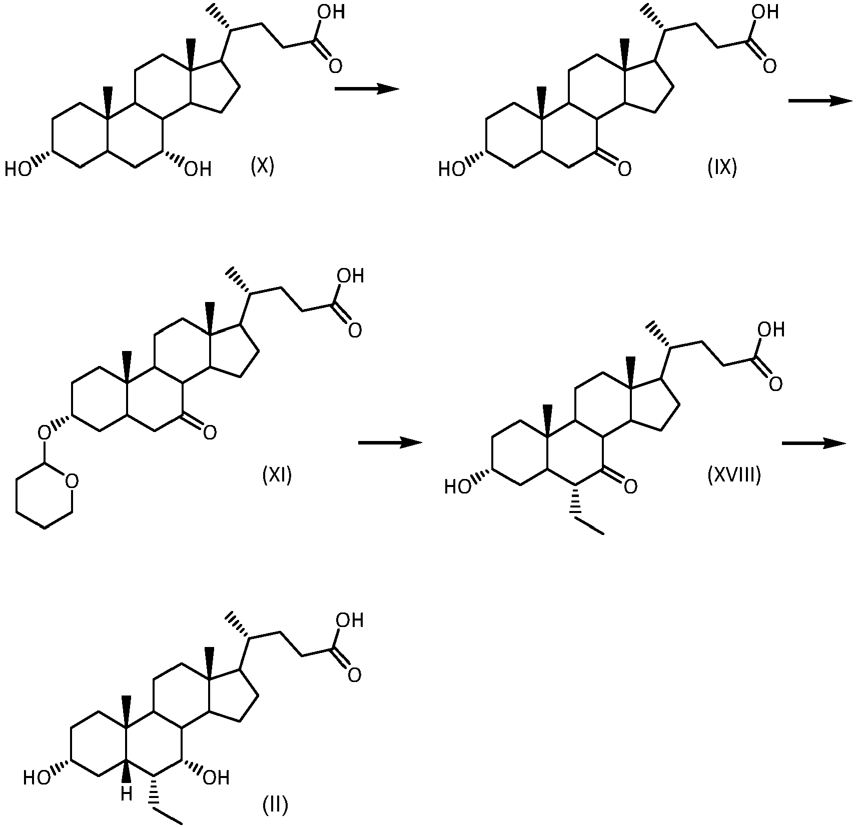

Nevertheless, this synthesis pathway also includes several purification steps by column chromatography, so it is not suitable for industrial implementation.

CN 107400154 A discloses the synthesis method shown in the scheme below for obtaining obeticholic acid, wherein R is C₁-C₆ alkyl:

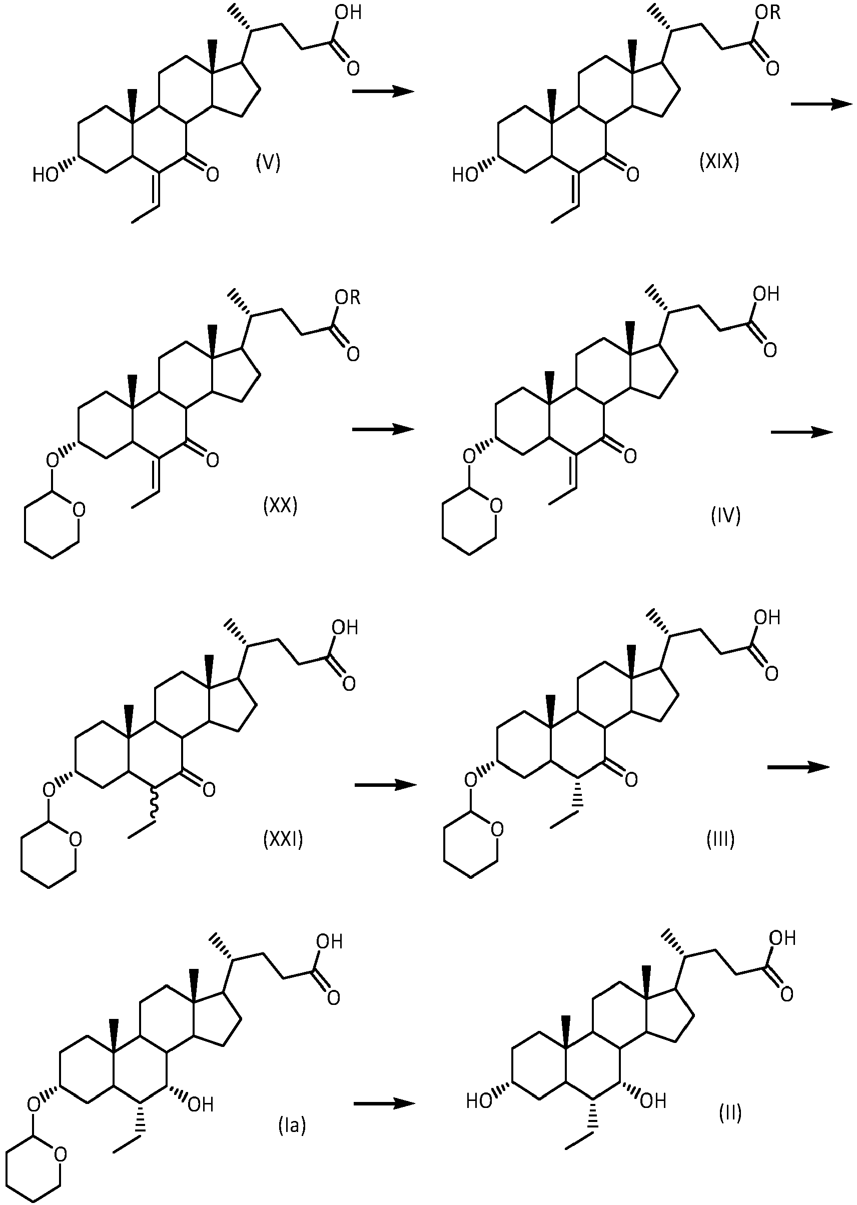

Similarly to the preceding method, CN 106589039 A also discloses a synthesis method of obeticholic acid wherein intermediates having a methyl ester as the carboxylic acid protecting group are used and/or a tetrahydropyranyl (THP) is used as the hydroxyl protecting group at the C3-position.

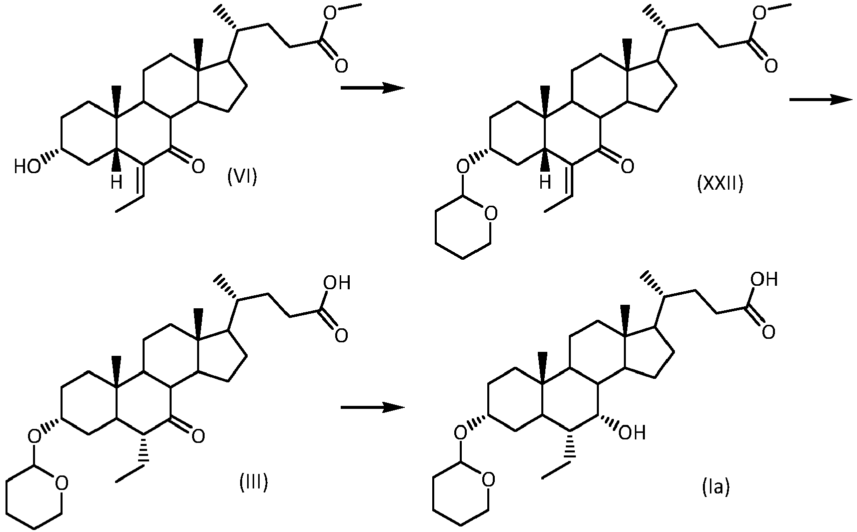

In the synthetic methods described in CN 107400154 A and CN 106589039 A, the obtained intermediates in each of the steps are isolated, which is a drawback for carrying out the method in an industrial manner.

CN 107955058 A discloses a synthesis method of obeticholic acid comprising the steps shown in the scheme below, said method comprising a series of steps for obtaining the precursor of formula (Ia). Deprotection of the tetrahydropyranyl group in the precursor of formula (Ia) renders obeticholic acid.

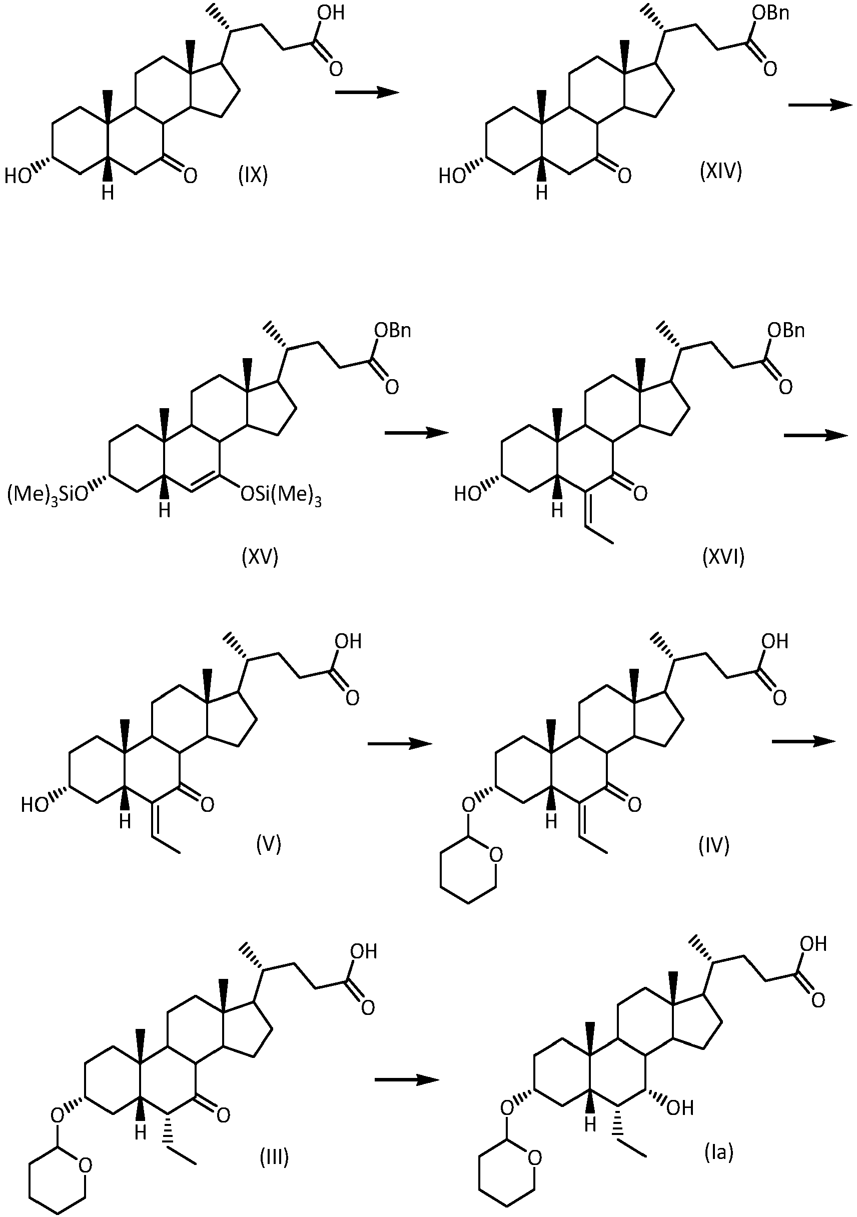

The method described in CN 107955058 A mentions the possibility of purifying the compound of formula (V) by the formation of an amine salt, particularly cyclohexylamine, diisopropylamine, or triethylamine salt. The formation of salts would represent an advantage in terms of the purification of intermediates in methods on an industrial scale. However, similarly to the preceding methods, in the method described in CN 107955058 A the obtained intermediates in each of the steps are also isolated, which is a drawback for carrying out the method in an industrial manner.

Therefore, there is a need in the state of the art for alternative methods for the synthesis of obeticholic acid which present improvements with respect to those already in existence, for example improvements in terms of the yield, purity, number of independent steps involving isolation of the obtained intermediates, and/or purity of obeticholic acid.

### Summary of the Invention

The inventors have discovered a new salt of a synthesis intermediate of obeticholic acid, the diethylamine salt of 3α-tetrahydropyranyloxy-6α-ethyl-7α-hydroxy-5β-cholanic acid, which can be obtained with a higher yield and/or purity and/or presents greater stability in relation to other salts of 3α-tetrahydropyranyloxy-6α-ethyl-7α-hydroxy-5β-cholanic acid, which are relevant in the manufacture of pharmaceutical products. Another advantageous feature of the diethylamine salt of 3α-tetrahydropyranyloxy-6α-ethyl-7α-hydroxy-5β-cholanic acid is that, in the event of wanting to further increase the purity level of the salt of 3α-tetrahydropyranyloxy-6α-ethyl-7α-hydroxy-5β-cholanic acid, the diethylamine salt of said acid can be readily purified by recrystallization. Furthermore, the use of the diethylamine salt of 3α-tetrahydropyranyloxy-6α-ethyl-7α-hydroxy-5β-cholanic acid allows obtaining obeticholic acid with high yields and purity levels. Arriving at this intermediate salt with a high purity allows obtaining obeticholic acid with the desired purity. Another advantage associated with the new diethylamine salt of 3α-tetrahydropyranyloxy-6α-ethyl-7α-hydroxy-5β-cholanic acid of the present invention is that it can be obtained from 3α-hydroxy-6α-ethylidene-7-keto-5β-cholanic acid (the compound of formula (V)) in a single reaction vessel and without the need to isolate the synthesis intermediates. The inventors thereby reduce the number of independent steps involving isolation of the obtained intermediates as well as the corresponding purification steps, achieving good yields and a high purity.

Thus, in a first aspect the present invention relates to the diethylamine salt of 3α-tetrahydropyranyloxy-6α-ethyl-7α-hydroxy-5β-cholanic acid of formula (I).

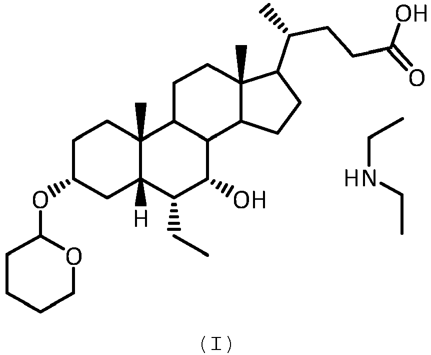

In a second aspect, the present invention relates to the use of the diethylamine salt of 3α-tetrahydropyranyloxy-6α-ethyl-7α-hydroxy-5β-cholanic acid of formula (I) in a method for the preparation of obeticholic acid of formula (II), particularly wherein the method for the preparation of obeticholic acid comprises treating the diethylamine salt of 3α-tetrahydropyranyloxy-6α-ethyl-7α-hydroxy-5β-cholanic acid of formula (I) with an acid at a pH between 0 and 3 to yield the obeticholic acid of formula (II).

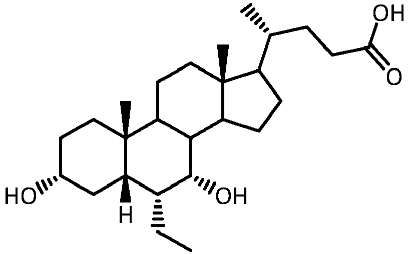

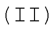

In a third aspect, the present invention relates to a method for the preparation of the diethylamine salt of 3α-tetrahydropyranyloxy-6α-ethyl-7α-hydroxy-5β-cholanic acid of formula (I), which method comprises treating 3α-tetrahydropyranyloxy-6α-ethyl-7α-hydroxy-5β-cholanic acid of formula (Ia) with diethylamine.

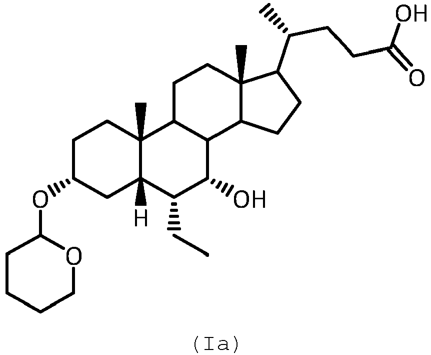

### Description of the Drawings

Figure 1 shows the X-ray powder diffraction (XRPD) pattern of the diethylamine salt of formula (I) obtained in Example 7.
Figure 2 shows the differential scanning calorimetry (DSC) graph of the diethylamine salt of formula (I) obtained in Example 7.
Figure 3 shows the X-ray powder diffraction (XRPD) pattern of the obeticholic acid of formula (II) obtained in Example 8.
Figure 4 shows the differential scanning calorimetry (DSC) graph of the obeticholic acid of formula (II) obtained in Example 8.
Figure 5 shows the differential scanning calorimetry (DSC) graph of the trometamol salt obtained in Comparative Example 9.2.
Figure 6 shows the differential scanning calorimetry (DSC) graph of the glycine salt obtained in Comparative Example 9.3.
Figure 7 shows the differential scanning calorimetry (DSC) graph of the taurine salt obtained in Comparative Example 9.4.
Figure 8 shows the differential scanning calorimetry (DSC) graph of the triethylamine salt obtained in Comparative Example 9.5.

### Detailed Description of the Invention

### Diethylamine salt of formula (I)

The present invention relates to amine salts of 3α-tetrahydropyranyloxy-6α-ethyl-7α-hydroxy-5β-cholanic acid, particularly to diethylamine salt of 3o;-tetrahydropyranyloxy-6α-ethyl-7α-hydroxy-5β-cholanic acid of formula (I).

The first aspect of the present invention relates to the diethylamine salt of 3α-tetrahydropyranyloxy-6α-ethyl-7α-hydroxy-5β-cholanic acid of formula (I).

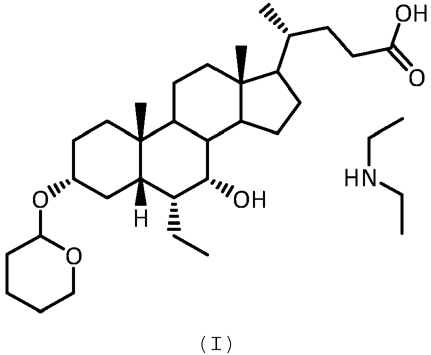

In the context of the present invention, the term "salt" must be understood to mean any form of an acid which assumes an ionic form or is charged (an anion), such as a carboxylic acid anion or carboxylate, and is coupled with a counterion (a cation), such as diethylamine (diethylammonium), triethylamine (triethylammonium), ammonium (NH₄⁺), sodium (Na⁺), or potassium (K⁺) cation, among others. "Amine salts" refer to salts in which the cation comes from a primary amine (⁺NH₂R), a secondary amine (⁺NHRR'), or a tertiary amine (⁺NRR'R").

In a preferred embodiment, diethylamine and 3α-tetrahydropyranyloxy-6α-ethyl-7α-hydroxy-5β-cholanic acid are at an approximate molar ratio of 1:1, i.e., for every mol of diethylamine present in the salt there is one mole of 3α-tetrahydropyranyloxy-6α-ethyl-7α-hydroxy-5β-cholanic acid.

In a preferred embodiment, the diethylamine salt of 3α-tetrahydropyranyloxy-6α-ethyl-7α-hydroxy-5β-cholanic acid of formula (I) is in solid form.

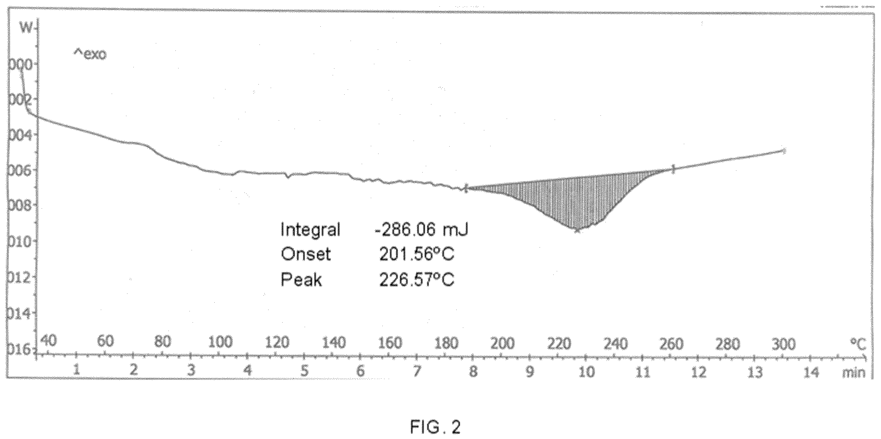

In another preferred embodiment, the diethylamine salt of 3α-tetrahydropyranyloxy-6α-ethyl-7α-hydroxy-5β-cholanic acid of formula (I) of the present invention is characterized by having a differential scanning calorimetry (DSC) graph comprising an endothermic peak having an onset temperature of about 202°C ± 0.3°C, particularly, the diethylamine salt of formula (I) has a differential scanning calorimetry graph substantially as shown in Figure 2. Particularly, the diethylamine salt of formula (I) of the present invention is characterized by having a differential scanning calorimetry graph comprising an endothermic peak between 170 and 270°C. Particularly, the diethylamine salt of formula (I) of the present invention is characterized by having a differential scanning calorimetry graph comprising an endothermic peak with a peak temperature between 220 and 230°C. The differential scanning calorimetry graph can be obtained as described in the Examples.

The "onset temperature" or "T onset" refers to the temperature resulting from extrapolating the baseline before the start of the transition and the baseline during energy absorption (tangent to the energy absorption curve (peak) at the inflection point) . It can be calculated as defined in standard DIN EN ISO 11357-1:2016(E).

The "peak temperature" refers to the temperature at which there is a greater distance between the curve of the peak to which it refers and the baseline resulting from extrapolating the baseline before the start of the transition.

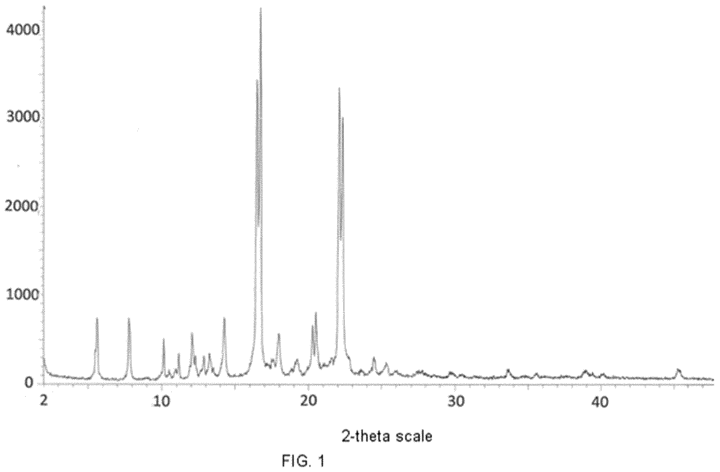

In another preferred embodiment, the diethylamine salt of 3α-tetrahydropyranyloxy-6α-ethyl-7α-hydroxy-5β-cholanic acid of formula (I) of the present invention is characterized by being in a crystalline form, the X-ray powder diffraction pattern of which has peaks at 5.5, 7.8, 10.1, 11.1, 12.1, 12.8, 13.2, 14.2, 16.4, 16.7, 17.9, 20.3, 20.5, 22.1, and 23.3°2Θ ± 0.2°2θ. Particularly, the diethylamine salt of 3α-tetrahydropyranyloxy-6α-ethyl-7α-hydroxy-5β-cholanic acid of formula (I) of the present invention has an X-ray powder diffraction pattern substantially as shown in Figure 1. The X-ray diffraction patterns can be recorded using a powder diffraction system with a copper anode emitting CuKα radiation with a wavelength of 1.54 Å, particularly by following the method described in the Examples.

### Use of the diethylamine salt of formula (I) for obtaining obeticholic acid

The present invention also relates to the use of the amine salts of 3α-tetrahydropyranyloxy-6α-ethyl-7α-hydroxy-5β-cholanic acid defined above in a method for the preparation of obeticholic acid, particularly wherein the amine salt is the diethylamine salt of formula (I).

The second aspect of the present invention relates to the use of the diethylamine salt of formula (I) in a method for the preparation of obeticholic acid, preferably wherein the method for the preparation of obeticholic acid comprises treating the diethylamine salt of formula (I) with an acid at a pH of 0 to 3 to yield the obeticholic acid of formula (II)

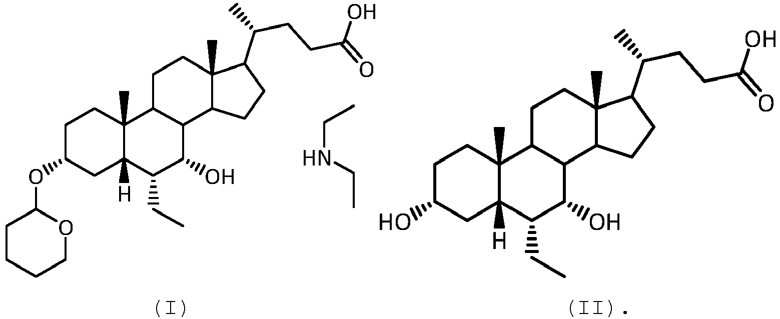

Determining the pH value, for example by using a pH meter, is part of the regular routine of the one skilled in the art.

Obtaining obeticholic acid (compound of formula (II)) by deprotection of the tetrahydropyranyl group in the diethylamine salt of formula (I) comprises treating the diethylamine salt of formula (I) with an acid at a pH of 0 to 3.

Any acid suitable for the deprotection of hydroxyl groups protected with a tetrahydropyranyl group can be used.

Examples of suitable acids are hydrochloric acid, p-toluenesulfonic acid, sulfuric acid, phosphoric acid, and methanesulfonic acid, among others. Preferably, the acid is hydrochloric acid.

Particularly, between 2 and 4 mol, more preferably between 2 and 3 mol, more preferably between 2 and 2.5 mol, even more preferably about 2.25 mol of acid are used for every mol of diethylamine salt of formula (I).

In a preferred embodiment, the treatment with an acid is performed in the presence of a solvent selected from the group consisting of C₁-C₄ alcohols, ketones, C₁-C₄ alkyl acetates, cyclic or linear ethers, acetonitrile, water, and mixtures thereof. Examples of C₁-C₄ alcohols are methanol, ethanol, n-propanol, isopropanol, n-butanol, isobutanol, sec-butanol, and tert-butanol, preferably methanol. Examples of ketones are acetone and methyl-*iso*-butylketone. Examples of C₁-C₄ alkyl acetates are ethyl acetate, n-butyl acetate, and tert-butyl acetate. Examples of cyclic or linear ethers are diethyl ether, tetrahydrofuran, and dioxane. In a more preferred embodiment, the solvent is a mixture of methanol and water. Preferably, between 5 and 15 ml, more preferably between 8 and 12 ml, even more preferably about 10 mL of methanol are used for every gram of diethylamine salt of formula (I).

In another preferred embodiment, the treatment with an acid is performed at a temperature between 15°C and 35°C, more preferably between 15°C and 30°C, more preferably between 20°C and 25°C.

In a particular embodiment, the treatment with an acid has a duration between 6 and 10 hours, preferably between 7 and 9 hours, more preferably about 8 hours.

After the treatment with an acid, the obeticholic acid of formula (II) is obtained, except in the case of having used a C₁-C₄ alcohol as a solvent (such as methanol, for example), in which case the corresponding ester of obeticholic acid and the C₁-C₄ alcohol used (for example obeticholic acid methyl ester) is obtained. The obeticholic acid can be isolated by the usual methods known to one skilled in the art.

In a particular embodiment, for isolating the obeticholic acid obtained after the treatment with an acid, the resulting mixture is basified, preferably to a pH of at least 11, preferably a pH of about 12. This step is essential in the event of having used a C₁-C₄ alcohol (for example methanol) as a solvent in the acid treatment of the diethylamine salt (I), for hydrolyzing the corresponding ester (for example the obeticholic acid methyl ester). Any base suitable for having the diethylamine in the form of a free base can be used, optionally hydrolyzing the ester (in the event of having used a C₁-C₄ alcohol, preferably methanol, as a solvent in the treatment of the diethylamine salt (I) with acid), and forming a salt between the base cation and the carboxylate anion. Particularly, it can be treated with a basic aqueous solution of sodium hydroxide or potassium hydroxide, preferably an aqueous solution of sodium hydroxide. Particularly, this treatment with a base is performed at a temperature between 15°C and 60°C, more preferably between 30°C and 60°C, more preferably between 45°C and 55°C. In a particular embodiment, this treatment with a base has a duration between 2 and 8 hours, preferably between 4 and 6 hours, more preferably about 5 hours. After this base treatment, at least 70%, preferably at least 75%, more preferably at least 80%, more preferably at least 85%, more preferably at least 90%, even more preferably at least 95% of the volume of the solvent present in the medium can be removed. This solvent includes the free diethylamine which has been formed after the addition of the base, such that the removal of the solvent also implies the removal of the free diethylamine from the obtained mixture. The removal of the solvent (including the free diethylamine) is preferably performed by distillation, particularly by distillation at a temperature of less than 40°C, more preferably of about 35°C. Particularly, said distillation is performed at reduced pressure, i.e., at a pressure of less than 101,325 Pa (1 atm), and which is capable of removing the solvent at a temperature of less than 40°C or 35°C. This pressure is readily determined by the one skilled in the art. After removing the solvent, the obtained mixture comprising obeticholic acid in a salt form is acidulated. Any acid suitable for forming free obeticholic acid can be used, such as, for example, the acids described above, such as hydrochloric acid, p-toluenesulfonic acid, sulfuric acid, phosphoric acid, and methanesulfonic acid, among others; preferably the acid is hydrochloric acid. Particularly, it can be treated with an acid at a pH less than or equal to 2, more preferably at a pH less than or equal to 1. Particularly, it can be treated with an aqueous solution of hydrochloric acid. The treatment can be performed at a temperature between 15°C and 35°C, more preferably between 15°C and 30°C, more preferably between 20°C and 25°C. Preferably, the obtained product is isolated by filtration.

### Method for the preparation of the diethylamine salt of formula (I)

The present invention also relates to a method for the preparation of the amine salts of 3α-tetrahydropyranyloxy-6α-ethyl-7α-hydroxy-5β-cholanic acid defined above, particularly the diethylamine salt of formula (I), which method comprises treating 3α-tetrahydropyranyloxy-6α-ethyl-7α-hydroxy-5β-cholanic acid (Ia) with the corresponding amine.

In a third aspect, the present invention relates to a method for the preparation of the diethylamine salt of formula (I), which method comprises treating 3α-tetrahydropyranyloxy-6α-ethyl-7α-hydroxy-5β-cholanic acid (Ia) with diethylamine.

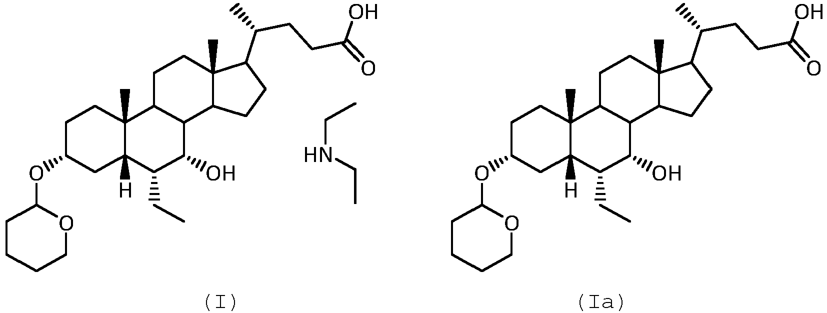

In a preferred embodiment, the treatment of 3α-tetrahydropyranyloxy-6α-ethyl-7α-hydroxy-5β-cholanic acid (Ia) with diethylamine is performed in the presence of a solvent selected from the group consisting of C₁-C₄ alkyl acetates, C₁-C₄ alcohols, ketones, water, and mixtures thereof, preferably ethyl acetate. Examples of said solvents have been previously defined. Determining the amount of solvent suitable for the reaction is part of the usual practice of the one skilled in the art. Particularly, a volume of solvent in which 3α-tetrahydropyranyloxy-6α-ethyl-7α-hydroxy-5β-cholanic acid (Ia) is in a solution is used. In a particular embodiment, at least 2 mL of solvent are used for every mmol of 3α-tetrahydropyranyloxy-6α-ethyl-7α-hydroxy-5β-cholanic acid (Ia), preferably between 2 and 3 mL of solvent for every mmol of acid (Ia).

In a particular embodiment, the treatment of 3α-tetrahydropyranyloxy-6α-ethyl-7α-hydroxy-5β-cholanic acid (Ia) with diethylamine is performed at a temperature between 15°C and 80°C, more preferably between 20°C and 70°C, more preferably between 15°C and 40°C, more preferably between 25°C and 45°C.

In a particular embodiment, the treatment of 3α-tetrahydropyranyloxy-6α-ethyl-7α-hydroxy-5β-cholanic acid (Ia) with diethylamine is maintained from 0.5 to 20 hours, preferably between 1 and 10 hours.

In a particular embodiment, the obtained diethylamine salt of formula (I) is subjected to a purification step, preferably recrystallization. A solvent suitable for recrystallization would be ethyl acetate, preferably 4 mL of ethyl acetate with respect to every gram of diethylamine salt of formula (I).

3α-Tetrahydropyranyloxy-6α-ethyl-7α-hydroxy-5β-cholanic acid (Ia) and the method of obtaining the same have been described in the state of the art, such as for example in CN 107400154 A, CN 106589039 A, and CN 107955058 A. In the present invention, preferably 3α-tetrahydropyranyloxy-6α-ethyl-7α-hydroxy-5β-cholanic acid (Ia) is obtained by a method which comprises:
(a) treating a compound of formula (III) or a salt thereof with a reducing agent to yield 3α-tetrahydropyranyloxy-6α-ethyl-7o;-hydroxy-5β-cholanic acid (Ia) or a salt thereof 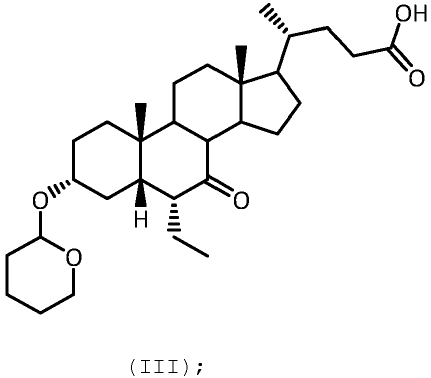 and
(b) optionally treating the salt of 3α-tetrahydropyranyloxy-6α-ethyl-7α-hydroxy-5β-cholanic acid (Ia) with an acid at a pH of 4 to 6 to yield 3α-tetrahydropyranyloxy-6α-ethyl-7α-hydroxy-5β-cholanic acid (Ia);
wherein the salt of the compound of formula (III) and the salt of 3α-tetrahydropyranyloxy-6α-ethyl-7α-hydroxy-5β-cholanic acid (Ia) are not diethylamine salts.

Preferably, in step (a) a salt of the compound of formula (III) is treated. The salt of the compound of formula (III) is formed by the carboxylic acid anion (carboxylate) and a cation. In a particular embodiment, the cation is selected from the group consisting of Na⁺, K⁺, and NH₄⁺, preferably Na⁺. In this method, the cation of the salt of the compound of formula (III) is not a diethylammonium cation, i.e., the salt of the compound of formula (III) is not a diethylamine salt.

The salt of 3α-tetrahydropyranyloxy-6α-ethyl-7α-hydroxy-5β-cholanic acid (Ia) is formed by the carboxylic acid anion of 3α-tetrahydropyranyloxy-6α-ethyl-7α-hydroxy-5β-cholanic acid (Ia) and a cation. In a particular embodiment, the cation is selected from the group consisting of Na⁺, K⁺, and NH₄⁺, preferably Na⁺. In this method, the cation of the salt of the acid (Ia) is not a diethylammonium cation, i.e., the salt of 3α-tetrahydropyranyloxy-6α-ethyl-7α-hydroxy-5β-cholanic acid is not a diethylamine salt.

In step (a) the ketone group at the C7-position is reduced to a hydroxyl group to yield 3o;-tetrahydropyranyloxy-6α-ethyl-7α-hydroxy-5β-cholanic acid (Ia) or a salt thereof. Reducing agents suitable for this step are those capable of reducing a ketone to hydroxyl, such as for example, sodium borohydride, lithium aluminum hydride, sodium triacetoxyborohydride, sodium cyanoborohydride, BH₃-(CH₃)₂S, and cerium trichloride, among others, preferably sodium borohydride.

Performing step (a) by treatment of a salt of the compound of formula (III) with sodium borohydride to yield a salt of 3α-tetrahydropyranyloxy-6α-ethyl-7α-hydroxy-5β-cholanic acid (Ia) is particularly advantageous and preferred.

In a preferred embodiment, step (a) is performed in the presence of a solvent selected from the group consisting of C₁-C₃ alcohol, water, and mixtures thereof. Examples of C₁-C₃ alcohols are methanol, ethanol, n-propanol, and isopropanol, preferably methanol. In a preferred embodiment, the solvent is a mixture of methanol and water. Preferably between 2 and 10 ml, more preferably between 3.5 and 6 mL of methanol are used for every mmol of compound of formula (III) or a salt thereof. Preferably between 2 and 10 ml, more preferably between 3.5 and 6 mL of methanol are used for every mmol of compound of formula (IV) or a geometric isomer thereof where the reaction of the compound of formula (V) or a geometric isomer thereof to yield the diethylamine salt of formula (I) is performed one pot, i.e. without isolating the intermediate compounds obtained.

In a preferred embodiment, between 1 and 1.5 mol, preferably between 1 and 1.2 mol, more preferably between 1 and 1.1 mol of reducing agent, preferably sodium borohydride, are used with respect to every mol of compound of formula (III) or a salt thereof.

In a preferred embodiment, step (a) is performed at a temperature between 20°C and 60°C, preferably between 25°C and 50°C, more preferably between 30°C and 45°C, even more preferably between 35°C and 45°C, even more preferably at about 40°C. In a particular embodiment, said temperature is maintained between 2.5 and 4 hours, preferably between 2.5 and 3.5 hours, more preferably about 3 hours.

In a particular embodiment, when the reducing agent in step (a) is sodium borohydride, a solution of sodium hydroxide in water is also added, preferably between 4 and 6 mol, preferably between 4 and 5 mol of sodium hydroxide are added with respect to every mol of sodium borohydride.

After step (a), optional step (b) for treating the salt of 3α-tetrahydropyranyloxy-6α-ethyl-7α-hydroxy-5β-cholanic acid (Ia) with an acid at a pH of 4 to 6 is performed to yield (free) 3α-tetrahydropyranyloxy-6α-ethyl-7α-hydroxy-5β-cholanic acid (Ia). This step (b) is performed when a salt of the acid (Ia) has been obtained after step (a).

Examples of acids suitable for step (b) are phosphoric acid, hydrochloric acid, acetic acid, sulfurous acid, and oxalic acid. Preferably, the acid used in step (b) is phosphoric acid, more preferably an aqueous solution of the acid, particularly an aqueous solution of phosphoric acid.

In a particular embodiment, the treatment with an acid of step (b) is performed at a pH of 4.5 to 5.5, more preferably about 5. Determining the pH value, for example by using a pH meter, is part of the regular routine of the one skilled in the art.

The compound of formula (III) and the method of obtaining the same have been described in the state of the art, such as for example in CN 107400154 A, CN 106589039 A, and CN 107955058 A. In the present invention, the compound of formula (III) or a salt thereof is preferably obtained by a method which comprises:
(a)treating a compound of formula (IV) or a geometric isomer thereof with a base and hydrogen and in the presence of a catalyst to yield a salt of the compound of formula (III) 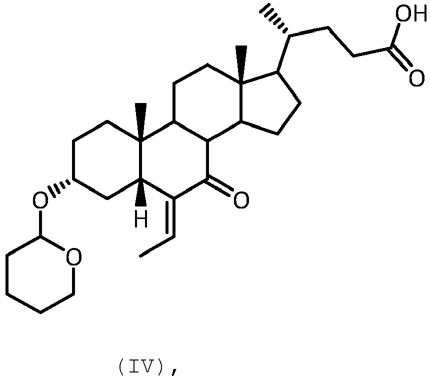 and
(b)optionally treating the salt of the compound of formula (III) with an acid at a pH of 4 to 6 to yield the compound of formula (III).

Preferably, a salt of the compound of formula (III) is obtained. In this case, step (b) is not performed. As described above, the salt of the compound of formula (III) is formed by the carboxylic acid anion (carboxylate) and a cation. In a particular embodiment, the cation is selected from the group consisting of Na⁺, K⁺, and NH₄⁺, preferably Na⁺. Said cation is not a diethylammonium cation, i.e., the salt of the compound of formula (III) is not a diethylamine salt.

In this preferred embodiment, the exocyclic double bond at the C6-position of a compound of formula (IV) or a geometric isomer thereof is reduced, and said carbon at the C6-position is epimerized to the alpha form (α), a salt of the compound of formula (III) thereby being obtained. These transformations are achieved by the treatment of the compound of formula (IV) or a geometric isomer thereof with a base and hydrogen and in the presence of a catalyst.

The term "geometric isomer" refers to stereoisomers differing only in the position of the substituents linked to a double bond, in the present case, the exocyclic double bond of the compounds of formula (IV). The possible geometric isomers are *cis* (*Z*) and *trans* (*E*)*.*

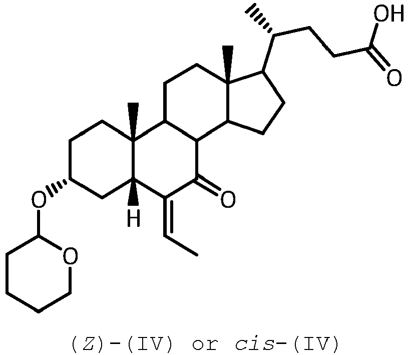

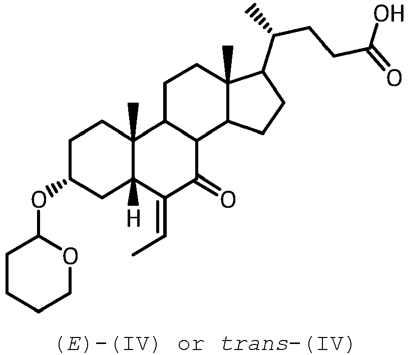

In the present invention, the compound of formula (IV) can be the Z isomer, E isomer, or a mixture of said isomers. Preferably, it is E isomer.

Examples of bases suitable for the treatment with hydrogen are sodium hydroxide, potassium hydroxide, ammonia, among others, preferably sodium hydroxide. Preferably, the base is mixed with water, more preferably the base is an aqueous solution, particularly an aqueous solution of sodium hydroxide.

In a preferred embodiment, between 1.5 and 2.5 mol, preferably between 1.5 and 2.1 mol, more preferably between 1.8 and 2.1 mol, more preferably between 1.9 and 2.1 mol, most preferably about 2 mol of base (preferably sodium hydroxide) are used with respect to every mol of compound of formula (IV) or a geometric isomer thereof.

In another preferred embodiment, the catalyst is selected from the group consisting of palladium on carbon, palladium on calcium carbonate, and platinum oxide, preferably the catalyst is palladium on carbon.

In a preferred embodiment, the treatment with hydrogen of step (a) is performed at a temperature between 15°C and 50°C, more preferably between 15°C and 45°C, more preferably between 20°C and 45°C, more preferably between 25°C and 45°C, more preferably between 30°C and 45°C, more preferably between 35°C and 45°C, preferably between 38°C and 42°C, more preferably about 40°C.

In another preferred embodiment, the treatment with hydrogen of step (a) is performed at a pressure between 4.5 and 5.5 bar, preferably between 4.5 and 5.2 bar, more preferably between 4.8 and 5.2 bar, even more preferably between 4.8 and 5.0 bar, most preferably about 5 bar.

In a particular embodiment, the treatment with hydrogen of step (a) is maintained between 4 and 10 hours, preferably between 4 and 8 hours, more preferably between 4 and 6 hours, most preferably about 5 hours.

In a preferred embodiment, the treatment with hydrogen of step (a) is performed in the presence of a solvent selected from a C₁-C₃ alcohol. Said solvent can also contain mixtures with water, particularly when the base used is in the form of an aqueous solution. Examples of C₁-C₃ alcohols are methanol, ethanol, n-propanol, and isopropanol, preferably methanol. In a preferred embodiment, between 2 and 10 ml, more preferably between 2 and 5 ml, even more preferably between 3 and 4 mL of C₁-C₃ alcohol are used for every mmol of compound of formula (IV) or a geometric isomer thereof.

Particularly, this step of treatment with hydrogen of step (a) is performed by first adding the base and the solvent to the compound of formula (IV) or a geometric isomer thereof; the resulting mixture is treated with activated carbon and subsequently the resulting mixture is filtered by any conventional method known to the one skilled in the art, for example, by a filter with diatomaceous earth, and hydrogen is then added.

In a particular embodiment, after hydrogenation the resulting mixture is filtered to remove the catalyst. Said filtration can be performed by any conventional method known to the one skilled in the art, for example, by a filter with diatomaceous earth. After this method, the compound of formula (III) is obtained in the form of a salt. In the event that the compound of formula (III) in the form of free acid is needed, the obtained salt of the compound of formula (III) can be treated with an acid at a pH of 4 to 6, i.e., step (b) is carried out.

Examples of acids suitable for this treatment of step (b) are phosphoric acid, hydrochloric acid, acetic acid, sulfurous acid, and oxalic acid. Preferably, the acid used in step (b) is phosphoric acid, more preferably an aqueous solution of the acid, particularly an aqueous solution of phosphoric acid.

In a particular embodiment, this treatment with an acid of step (b) is performed at a pH of 4.5 to 5.5, more preferably about 5. Determining the pH value, for example by using a pH meter, is part of the regular routine of the one skilled in the art.

The compound of formula (IV) and the method of obtaining the same have been described in the state of the art, such as for example in CN 107400154 A and CN 107955058 A. In the present invention, preferably the compound of formula (IV) or a geometric isomer thereof is obtained by treatment of a compound of formula (V) or a geometric isomer thereof with 3,4-dihydro-2H-pyran in the presence of an acid.

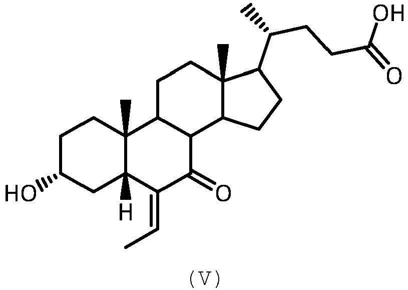

As defined with respect to the compound of formula (IV), geometric isomers refer to stereoisomers differing only in the position of the substituents linked to a double bond, in the present case, the exocyclic double bond of the compound of formula (V). The possible geometric isomers are *cis (Z)* and *trans (E).*

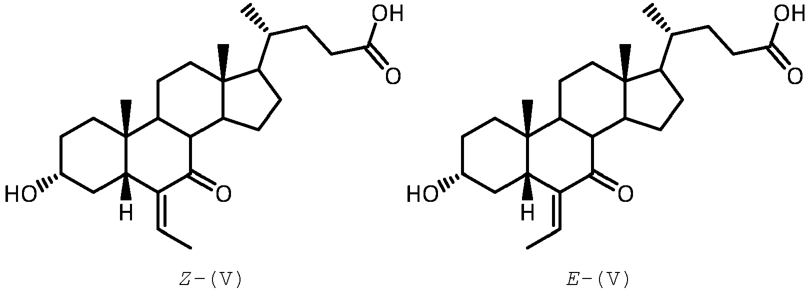

In the present invention, the compound of formula (V) can be isomer Z, isomer E, or a mixture of said isomers. Preferably, it is isomer E.

Any acid suitable for the protection of hydroxyl groups by the formation of tetrahydropyranyl ether can be used, such as for example camphorsulfonic acid and p-toluenesulfonic acid, among others. Preferably, the acid is camphorsulfonic acid, more preferably (1S)-(+)-10-camphorsulfonic acid.

In a preferred embodiment, between 0.04 and 0.06 mol, preferably between 0.05 and 0.06 mol, more preferably about 0.05 mol of acid are used with respect to every mol of compound of formula (V) or a geometric isomer thereof.

In another preferred embodiment, between 1 and 2 mol, preferably about 1.5 mol of 3,4-dihydro-2H-pyran are used with respect to every mol of compound of formula (V) or a geometric isomer thereof.

In another preferred embodiment, the treatment of the compound of formula (V) or a geometric isomer thereof with 3,4-dihydro-2H-pyran is performed in the presence of a solvent selected from the group consisting of dichloromethane, tetrahydrofuran, and mixtures thereof, preferably dichloromethane. Preferably, between 5 and 15 ml, more preferably about 10 mL of solvent are used with respect to every gram of compound of formula (V) or a geometric isomer thereof.

In another preferred embodiment, the treatment of the compound of formula (V) or a geometric isomer thereof with 3,4-dihydro-2H-pyran is performed at a temperature between 15°C and 35°C, preferably between 20°C and 25°C.

In a particular embodiment, the treatment of the compound of formula (V) or a geometric isomer thereof with 3,4-dihydro-2H-pyran has a duration between 1.5 and 10 hours, preferably between 1.5 and 3 hours, more preferably about 2 hours.

In another particular embodiment, said treatment is performed in inert atmosphere, for example in nitrogen or argon atmosphere.

In another particular embodiment, after the treatment of the compound of formula (V) or a geometric isomer thereof with 3,4-dihydro-2H-pyran the pH is adjusted to about 9.5, for example by adding a suitable base, such as triethylamine. Preferably, after adjusting the pH at least 70%, preferably at least 75%, more preferably at least 80%, more preferably at least 85%, more preferably at least 90%, even more preferably at least 95% of the volume of the solvent is removed. The removal of the solvent is preferably performed by distillation, particularly by distillation under reduced pressure, i.e., at a pressure of less than 101,325 Pa (1 atm), and which is capable of removing the solvent at the temperature used (this pressure is readily determined by the one skilled in the art), and preferably at a temperature of less than 40°C.

In a preferred embodiment of the invention, the treatment of the compound of formula (V) or a geometric isomer thereof to yield the amine salt of formula (I) is a one pot treatment, i.e., all the steps of the reaction (i.e., the treatment of the compound of formula (V) or a geometric isomer thereof with 3,4-dihydro-2H-pyran to yield the compound of formula (IV) or a geometric isomer thereof, the hydrogenation of said compound of formula (IV) or a geometric isomer thereof to yield the salt of the compound of formula (III), the treatment of said salt of formula (III) with sodium borohydride to yield the salt of 3α-tetrahydropyranyloxy-6α-ethyl-7α-hydroxy-5β-cholanic acid (Ia) and the subsequent treatment thereof with an acid to yield 3α-tetrahydropyranyloxy-6α-ethyl-7α-hydroxy-5β-cholanic acid (Ia), and finally the treatment of said acid (Ia) with diethylamine to yield the diethylamine salt of formula (I)) are performed in the same reaction vessel without the need to isolate the intermediate products obtained. This embodiment is particularly advantageous for the implementation thereof on an industrial scale and can further reduce the content of impurities of the amine salt of formula (I).

The compound of formula (V) has been described in the state of the art, such as for example in CN 107400154 A and CN 107955058 A. In a preferred embodiment of the present invention, the compound of formula (V) or a geometric isomer thereof is obtained by treatment of a compound of formula (VI) or a geometric isomer thereof in the presence of a base and a solvent selected from the group consisting of water, C₁-C₃ alcohol, and a mixture thereof.

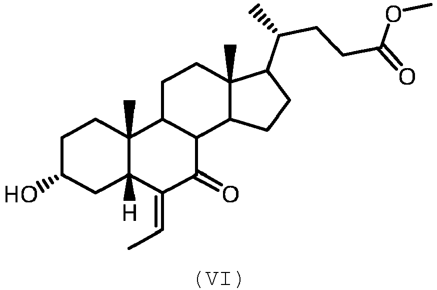

As defined with respect to the compound of formula (IV), geometric isomers refer to stereoisomers differing only in the position of the substituents linked to a double bond, in the present case, the exocyclic double bond of the compound of formula (VI). The possible geometric isomers are *cis (Z)* and *trans (E).*

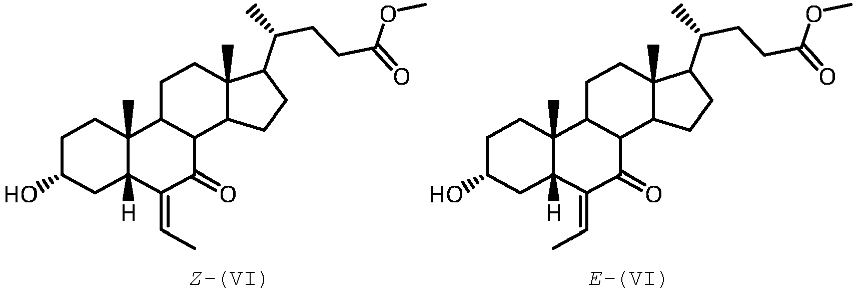

In the present invention, the compound of formula (VI) can be isomer Z, isomer E, or a mixture of said isomers. Preferably, it is isomer E.

This reaction step corresponds to the deprotection of the methyl ester (or saponification) of the compound of formula (VI) or a geometric isomer thereof. This reaction is well known to the one skilled in the art.

The base used in this step is any base suitable for carrying out the deprotection of methyl ester, such as for example sodium hydroxide or potassium hydroxide, among others, preferably sodium hydroxide. Preferably, the base is mixed with water, more preferably the base is an aqueous solution, particularly an aqueous solution of sodium hydroxide.

In a preferred embodiment, between 0.5 and 2.5 mol, preferably between 1 and 1.5 mol, more preferably between 1 and 1.1 mol of base (preferably sodium hydroxide) are used with respect to every mol of compound of formula (VI) or a geometric isomer thereof.

In a preferred embodiment, the solvent used in this deprotection is a mixture of water and C₁-C₃ alcohol. Examples of C₁-C₃ alcohols are methanol, ethanol, n-propanol, and isopropanol, preferably methanol. In a preferred embodiment, the solvent used in the deprotection is a mixture of water and methanol, preferably a mixture of water and methanol in a volumetric proportion of 1:1 to 1:10. When the base used is in the form of an aqueous solution, all or part of the water of the solvent described herein may come from said basic aqueous solution.

In one embodiment, said treatment of the compound of formula (VI) or a geometric isomer thereof is performed at a temperature between 40°C and 60°C, more preferably between 45°C and 55°C, more preferably about 50°C.

In a particular embodiment, after the treatment with a base the compound of formula (V) or a geometric isomer thereof can be isolated by removing the solvent from the reaction mixture, particularly by removing at least 70%, preferably at least 75%, more preferably at least 80%, more preferably at least 85%, more preferably at least 90%, even more preferably at least 95% of the volume of the solvent. The removal of the solvent is preferably performed by distillation, particularly by distillation at a temperature of less than 40°C and under reduced pressure (at a pressure of less than 101,325 Pa (1 atm) and capable of removing the solvent at a temperature of less than 40°C; this pressure is readily determined by the one skilled in the art). The obtained residue can be treated with an acid, particularly with aqueous hydrochloric acid, preferably by adjusting the pH of the resulting mixture to about 3. Particularly, after the acid treatment one or more washes can be performed with an organic solvent, such as for example a C₁-C₄ alkyl ester, preferably ethyl acetate. Preferably, after the washes at least 70%, preferably at least 75%, more preferably at least 80%, more preferably at least 85%, more preferably at least 90%, even more preferably at least 95% of the volume of the solvent is removed. The removal of the solvent is preferably performed by distillation, particularly by distillation under reduced pressure, i.e., at a pressure of less than 101,325 Pa (1 atm), and which is capable of removing the solvent at the temperature used (this pressure is readily determined by the one skilled in the art) and preferably at a temperature of less than 40°C.

In a preferred embodiment, the compound of formula (VI) or a geometric isomer thereof is obtained by treatment of a compound of formula (VII) with acetaldehyde in the presence of boron trifluoride-diethyl ether.

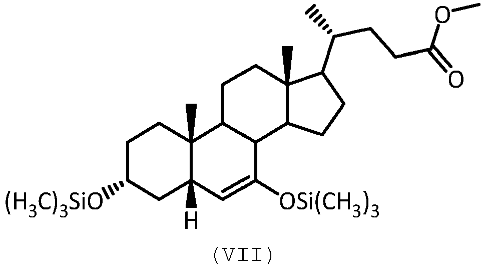

In a particular embodiment, between 1.5 and 5 mol, preferably between 2 and 4 mol, more preferably between 2.5 and 3.5 mol, even more preferably about 3 mol of acetaldehyde are used with respect to every mol of compound of formula (VII).

In another particular embodiment, between 1.5 and 5 mol, preferably between 2 and 4 mol, more preferably between 2.5 and 3.5 mol, even more preferably about 3 mol of boron trifluoride-diethyl ether are used with respect to every mol of compound of formula (VII).

In another particular embodiment, the treatment of the compound of formula (VII) with acetaldehyde in the presence of boron trifluoride-diethyl ether is performed in dichloromethane. Preferably, between 2 and 15 ml, more preferably between 7 and 15 mL of solvent with respect to every gram of compound of formula (VII) are used.

In another particular embodiment, the treatment of the compound of formula (VII) with acetaldehyde in the presence of boron trifluoride-diethyl ether is performed at a temperature between -60°C and -65°C, particularly for 1.5 to 3 hours, preferably for about 2 hours, followed by a temperature between 20°C and 25°C, particularly for 2 to 5 hours, preferably for about 3 hours.

In another particular embodiment, after the treatment of the compound of formula (VII) with acetaldehyde in the presence of boron trifluoride-diethyl ether one or more washes are performed with an aqueous solution of sodium bicarbonate. Preferably, after the washes at least 70%, preferably at least 75%, more preferably at least 80%, more preferably at least 85%, more preferably at least 90%, even more preferably at least 95% of the volume of the solvent is removed. The removal of the solvent is preferably performed by distillation, particularly by distillation under reduced pressure, i.e., at a pressure of less than 101,325 Pa (1 atm), and which is capable of removing the solvent at the temperature used (this pressure is readily determined by the one skilled in the art) and preferably at a temperature of less than 40°C.

In a preferred embodiment, the compound of formula (VII) is obtained by treatment of a compound of formula (VIII) with chlorotrimethylsilane or trimethylsilyl trifluoromethanesulfonate in the presence of a base.

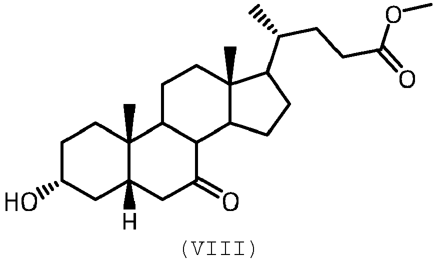

Bases suitable for this treatment are, for example, lithium diisopropylamide or mixtures of hexyl-lithium or n-butyl-lithium with diisopropylamine. Preferably, the compound of formula (VII) is obtained by treatment of a compound of formula (VIII) with chlorotrimethylsilane and lithium diisopropylamide. Preferably, between 4 and 5 mol, more preferably about 4.5 mol of chlorotrimethylsilane are used for every mol of compound of formula (VIII). Particularly, the treatment is performed in a suitable solvent, such as for example tetrahydrofuran, hexane, and mixtures thereof, preferably in a mixture of tetrahydrofuran and hexane. Preferably, between 5 and 25 ml, more preferably between 15 and 25 mL of solvent are used for every gram of compound of formula (VIII). Particularly, this treatment is performed in inert atmosphere (for example nitrogen or argon atmosphere) and preferably at a temperature between -70°C and -80°C. In a particular embodiment, after the treatment of the compound of formula (VIII) with chlorotrimethylsilane or trimethylsilyl trifluoromethanesulfonate in the presence of a base and a solvent, one or more washes are performed with an aqueous solution of citric acid. Preferably, after the washes at least 70%, preferably at least 75%, more preferably at least 80%, more preferably at least 85%, more preferably at least 90%, even more preferably at least 95% of the volume of the solvent is removed. The removal of the solvent is preferably performed by distillation, particularly by distillation under reduced pressure, i.e., at a pressure of less than 101,325 Pa (1 atm), and which is capable of removing the solvent at the temperature used (this pressure is readily determined by the one skilled in the art) and preferably at a temperature of less than 40°C.

In a preferred embodiment, the compound formula (VIII) is obtained by treatment of a compound of formula (IX) with methanol in the presence of an acid.

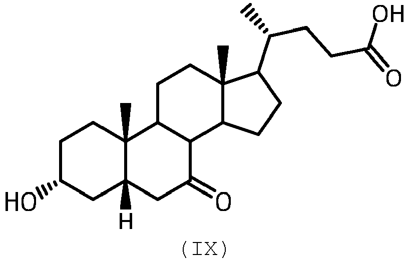

This step corresponds to the protection of the carboxylic acid group of the compound of formula (IX) in the form of methyl ester. This protection reaction is well known to the one skilled in the art.

Acids suitable for this treatment are any acid suitable for the protection of carboxylic acids by the formation of the corresponding methyl ester. Preferably, the acid is hydrochloric acid. Preferably, between 0.5 and 3 mol, more preferably between 1 and 2.5 mol, even more preferably between 2 and 2.5 mol of acid (preferably hydrochloric acid) are used for every mol of compound of formula (IX).

In a particular embodiment, between 5 and 15 ml, more preferably about 10 mL of methanol are used for every gram of compound of formula (IX).

Particularly, this treatment is performed for 1 to 5 hours, preferably for about 2 hours. Particularly, the treatment is performed at a temperature between 20°C and 25°C. In a particular embodiment, after the treatment of the compound of formula (IX) with methanol in the presence of an acid, the pH is adjusted to about 7, for example, by adding a suitable base, such as for example sodium hydroxide, particularly an aqueous solution of sodium hydroxide. Preferably, the compound of formula (VIII) is isolated by filtration.

The compound of formula (IX) and the method of obtaining the same have been described in the state of the art, such as for example in WO 02/072598 A1, US 83386258 B2, and CN 107955058 A. In the present invention, preferably the compound of formula (IX) is obtained by treatment of a compound of formula (X) with sodium bromide and sodium hypochlorite.

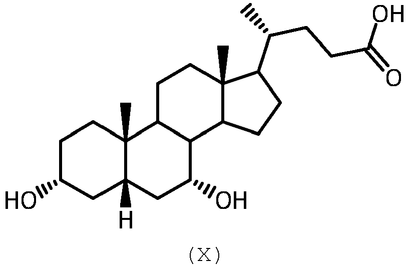

Preferably, between 0.05 and 0.15 mol, more preferably about 0.1 mol of sodium bromide are used for every mol of compound of formula (X). Preferably, 1 to 2 mol, more preferably 1.3 to 1.4 mol of sodium hypochlorite are used for every mol of compound of formula (X). Particularly, the treatment of the compound of formula (X) with sodium bromide and sodium hypochlorite is performed in the presence of acetic acid. Particularly, this treatment is performed for 10 to 20 hours, preferably for about 15 hours. Particularly, the treatment is performed at a temperature between -5°C and 5°C. In a particular embodiment, after this treatment of the compound of formula (X) water and sodium bisulfite are added. Preferably, the compound of formula (IX) is isolated by filtration.

The compound of formula (X) and the method of obtaining the same have been described in the state of the art, such as for example in Zampella et al. [J. Med. Chem., 2012, 55, 84-93], WO 02/072598 A1, US 83386258 B2, and CN 107955058 A.

In the context of the present invention, the terms "approximate" and "about" refer to the value which characterizes ±5% of said value.

In the context of the present invention, the term "acid" refers to a substance capable of donating a proton (to a base).

In the context of the present invention, the term "base" refers to a substance capable of accepting a proton (from an acid).

Several examples of the experimental methods and embodiments of the present invention are described below for the purpose of helping to comprehend the preceding ideas. Said examples are merely illustrative.

### Examples

### Materials and methods

XRPD analysis was performed in a Siemens D-500 model X-ray powder diffractometer equipped with a copper anode. Scanning parameters: 4-50 20 degrees, continuous scan, ratio: 1.2 35 degrees/minute.

DSC analysis was performed in a Mettler Toledo 822e apparatus with STARe SW15.00 software. Parameters: heating range of 25 to 300°C, except for obeticholic acid where a heating range of 25 to 200°C is applied, with a ramp of 5°C/min and N₂ flow of 50 mL/min. The measurement is taken with a perforated closed capsule.

The purity of the obtained products is analyzed by the high-performance liquid chromatography technique in a Waters Alliance apparatus, provided with refractive index detector and thermostatted oven for the column. The experimental conditions for obtaining a chromatogram were: Kinetex C18 2.6 um column (150 mm x 4.6 mm); mobile phase: 0.1% aqueous solution of phosphoric acid:methanol:acetonitrile (30:30:40); flow: 1.2 mL/min in isocratic mode; column temperature: 40°C; injection volume: 10 pL; solvent for the samples to be analyzed: water/methanol/acetonitrile (12:60:28); concentration: 1 mg/mL. Acquisition time: 45 min

¹³C and ¹H RMN spectra were acquired in a Bruker 400 spectrometer equipped with Prodigy cryoprobe.

### Example 1. Obtaining of 3o;-hydroxy-7-keto-5β-cholanic acid (compound of formula (IX))

250 g (636.8 mmol) of chenodeoxycholic acid (CDCA) and 6.55 g (63.7 mmol, 0.1 molar eq.) of NaBr were mixed with 1750 mL of methanol under vigorous stirring so as to homogenize the mixture. 34 mL (534.9 mmol, 1.05 molar eq.) of 90% acetic acid were subsequently added, and the resulting mixture was cooled at the temperature between -5 and 5°C. While maintaining said temperature, 371 mL of a 15% NaClO₄ solution (titration 164.55 g Cl₂/L, 1.35 molar eq.) were slowly added. The resulting reaction mixture was maintained under stirring for about 15 h at the temperature between -5 and 5°C. Once this maintenance was finished, the resulting mixture was heated at the approximate temperature of 25°C and 60 mL of an aqueous solution of 5% sodium bisulfite were slowly loaded. The obtained mixture was stirred for 30 minutes at the indicated temperature. 250 mL of water at the indicated temperature were added, and the obtained mixture was stirred for 30 minutes. The reaction mass was filtered and washed with 125 mL of methanol and two fractions of 250 mL of water each, and the solid thus obtained was dried in an air oven to constant weight, rendering 191.4 g (76.9% yield) of 3α-hydroxy-7-keto-5β-cholanic acid (IX).

### Example 2. Obtaining of methyl 3α-hydroxy-7-keto-5β-cholanate (compound of formula (VIII))

80 g (0.205 mmol) of 3α-hydroxy-7-keto-5β-cholanic acid (IX) were mixed with 800 mL of methanol. 38 mL (0.471 mol, 2.33 molar eq.) of an aqueous solution of 37% HCl were subsequently added to the resulting mixture, maintaining the temperature between 20 and 25°C. The reaction mixture was maintained at the mentioned temperature for 2 hours.

Once this maintenance was finished, an aqueous solution of 2N sodium hydroxide was slowly added to a pH value of about 7. 1000 mL of water at the temperature between 10 and 15°C were added, the obtained mixture was maintained for 30 minutes at the mentioned temperature, and subsequently the precipitated solid was filtered to obtain 83.2 g (99.7% yield) of a white solid corresponding to methyl 3α-hydroxy-7-keto-5β-cholanate (VIII). The purity of the obtained product analyzed by HPLC is 99.0%.

### Example 3. Obtaining of methyl 3α,7-bis(trimethylsilyloxy)-5β-cholan-6-enate (compound of formula (VII))

600 mL of tetrahydrofuran were cooled at about -65°C under nitrogen atmosphere, and 556 mL of a solution of lithium isopropylamide in 2 M hexane (1.112 mol, 6 molar eq.) were slowly added maintaining the indicated temperature. The obtained mixture was cooled at the temperature of about -72°C, and 106 mL (0.834 mol, 4.5 molar eq.) of TMSCl (chlorotrimethylsilane) were slowly added. A previously prepared solution of 75 g (0.185 mol) of methyl 3α-hydroxy-7-keto-5β-cholanate (VIII) in 375 mL of tetrahydrofuran was subsequently added to the obtained mixture. The mixture was maintained under stirring under nitrogen atmosphere at the temperature of about -72°C. Once this maintenance was finished, 100 mL of a previously prepared solution of 42.739 g (390 mmol) of citric acid in 375 mL of water was slowly added without the temperature exceeding 5°C. Once the addition was finished, the temperature of the mixture was left to reach 20°C and the organic phase was separated. The solvent was vacuum distilled until obtaining an oily residue corresponding to methyl 3α,7-bis(trimethylsilyloxy)-5β-cholan-6-enate (VII), which was used in the next synthesis step without further purification.

### Example 4. Obtaining of methyl 3o;-hydroxy-6-ethylidene-7-keto-5β-cholanate (compound of formula (VI))

101.5 g (185 mmol) of methyl 3α,7-bis(trimethylsilyloxy)-5β-cholan-6-enate (VII) were dissolved in 914 mL of dichloromethane and the resulting solution was cooled at the temperature between -60 and -65°C. 24.45 g (555 mmol, 3 molar eq.) of acetaldehyde and subsequently 78.77 g (555 mmol, 3 molar eq.) of boron trifluoride-diethyl ether were added. The reaction mixture was maintained under stirring for 2 hours at the temperature between -60 and -65°C and subsequently for 3 hours at the temperature between 20 and 25°C. Once this maintenance was finished, the resulting reaction mixture was added to 1320 mL of an 8% by weight aqueous solution of NaHCOs, and the resulting mixture was maintained under stirring for 30 minutes at the temperature between 25 and 30°C. The organic phase was separated, and the solvent was vacuum distilled so as to obtain a dense oil containing methyl 3α-hydroxy-6-ethylidene-7-keto-5β-cholanate (VI), which was used in the next synthesis step without further purification.

### Example 5. Obtaining of 3o;-hydroxy-6-ethylidene-7-keto-5β-cholanic acid (compound of formula (V))

37.5 g (87 mmol) of methyl 3o;-hydroxy-6-ethylidene-7-keto-5β-cholanate (VI) were dissolved in 53 mL of MeOH and 7.5 mL of H₂O and 7.5 mL of 50% sodium hydroxide were subsequently added. The mixture thus obtained was heated at the temperature of about 50°C and was maintained under stirring at said temperature for 2 h.

Once this maintenance was finished, the reaction solvent was vacuum distilled, and 150 mL of water were added to the resulting residue. The solution thus obtained was washed 3 times with 50 mL of t-butyl methyl ether, with the organic phases obtained in each wash being discarded. A 2N aqueous solution of HCl was added to a pH of about 3. The aqueous phase was washed 3 times with 50 mL of ethyl acetate. The organic phases from the washes thus obtained were pooled and the solvent was vacuum distilled. Two successive series of loading 100 mL of ethyl acetate and vacuum distillation were performed. Finally, 35 mL of ethyl acetate were added to the obtained residue, and the obtained suspension is filtered to render, after being dried in an air oven, 16.53 g of 3α-hydroxy-6-ethylidene-7-keto-5β-cholanic acid (V) with a purity analyzed by HPLC of 95.52%.

### Example 6. Obtaining of 3o;-tetrahydropyranyloxy-6-ethylidene-7-keto-5β-cholanic acid (compound of formula (IV))

16.0 g (38.4 mmol) of 3α-hydroxy-6-ethylidene-7-keto-5β-cholanic acid (V) and 0.45 g (1.9 mmol, 0.05 molar eq.) of (1S)-(+)-10-camphorsulfonic acid were mixed with 160 mL of dichloromethane under nitrogen atmosphere so as to obtain a solution at the temperature between 20 and 25°C. While maintaining said temperature, 5.3 mL (57.6 mmol, 1.5 molar eq.) of 3,4-dihydro-2H-pyran were added, and the resulting reaction mixture was maintained under stirring at the same temperature for 2 hours. Once this maintenance was finished, the pH of the reaction mixture was adjusted to an approximate value of 9.5 by adding triethylamine at the temperature between 20 and 25°C. The resulting reaction mixture was concentrated by vacuum distilling the solvent, and a very dense, virtually colorless oil corresponding to 3α-tetrahydropyranyloxy-6-ethylidene-7-keto-5β-cholanic acid (IV) was obtained. The purity of the obtained product analyzed by HPLC is 98.76%.

### Example 7. Obtaining of the diethylamine salt of 3α-tetrahydropyranyloxy-6α-ethyl-7α-hydroxy-5β-cholanic acid (compound of formula (I))

The oil obtained in the preceding example corresponding to 3α-tetrahydropyranyloxy-6-ethylidene-7-keto-5β-cholanic acid (IV) (38.4 mmol) was dissolved in 100 mL of methanol and 10.5 mL of a 30% aqueous solution (w/v) of sodium hydroxide (78.75 mmol, 2 molar eq.). 0.95 g of type 4S activated carbon were loaded, and the resulting mixture was heated to the temperature of about 40°C, maintaining the mixture under stirring for 30 minutes at said temperature. The mixture was subsequently cooled at the temperature of about 25°C. The mixture was filtered through a diatomaceous earth filter and the filtrate was washed with two fractions of 15 mL of methanol each. The combined filtrate and wash was introduced in a reactor under pressure, and 1.73 mg of 5% Pd/C were added. The reactor was pressurized with hydrogen to an internal pressure of 5 bar, and the resulting reaction mixture was maintained at the temperature of about 40°C for 5 hours. Once said maintenance was finished, the reaction mass was cooled at a temperature of about 20°C, and the Pd/C was filtered by a filter with diatomaceous earth, the filter being washed with two fractions of 25 mL of methanol each. The reaction mass was distilled under reduced pressure without exceeding the temperature of 40°C until obtaining a distillate volume of about 35 mL.

13.3 mL of a 14 M basic solution of sodium hydroxide and 12% of NaBH₄ (42.2 mmol of NaBH₄, 1.1 molar eq.) were slowly added to the obtained reaction mass at a temperature of about 40°C. The resulting reaction mixture was maintained under stirring at the reflux temperature for 3 hours (control by HPLC of the reaction mass reveals the complete conversion of the product 3α-tetrahydropyranyloxy-6α-ethyl-7-keto-5β-cholanic acid).

Once this maintenance was finished, the solvent of the reaction was vacuum distilled and without exceeding the temperature of 45°C. 40 mL of water and 40 mL of n-heptane were added to the obtained mass, and the resulting mixture was heated under stirring at the temperature of about 45°C. The stirring was stopped, the obtained mixture was maintained standing, and the resulting phases were separated. 40 mL of ethyl acetate were added to the obtained aqueous phase, and an 85% aqueous solution of phosphoric acid was added to a final pH of the mixture of about 5. The mixture was left to stand, and the phases thus obtained were separated. 40 mL of ethyl acetate were added to the aqueous phase, and the resulting phases were separated. The two organic phases thus obtained were pooled together and mixed with a volume of diethylamine until achieving a final pH of the mixture of about 8.5. A volume of about 75 mL of ethyl acetate was vacuum distilled and without exceeding the temperature of 45°C, and two successive series of loading 15 mL of ethyl acetate and vacuum distillation of about 15 mL of ethyl acetate were performed. Finally, 15 mL of ethyl acetate were added, and the resulting mixture was slowly cooled to the temperature of about 0°C. The resulting mixture was maintained at said temperature for 2 hours and the solid present was filtered and washed twice with 10 mL of ethyl acetate. The white solid thus obtained rendered, after being dried, 17.8 g (80.2%) corresponding to the diethylamine salt of 3α-tetrahydropyranyloxy-6α-ethyl-7α-hydroxy-5β-cholanic acid (I). The purity of the product was analyzed by HPLC, obtaining 99.77%. Figure 1 shows the XRPD of the obtained product and Figure 2 shows its DSC.

If desired, the diethylamine salt of 3α-tetrahydropyranyloxy-6α-ethyl-7α-hydroxy-5β-cholanic acid (I) can be recrystallized in ethyl acetate (4 volumes of solvent with respect to the total mass of the salt to be recrystallized). A typical example of purification by recrystallization allows obtaining the product with a recrystallization yield of about 95% and a purity of the product analyzed by HPLC of 99.97%.

Table 1 shows the data relating to the diethylamine salt of 3α-tetrahydropyranyloxy-6α-ethyl-7α-hydroxy-5β-cholanic acid (I) obtained from the ¹H-NMR (DMSO-d6, 400 MHz) and ¹³C-NMR (DMSO-d6, 100 MHz) experiments. The chemical shifts were in reference to the DMSO-d6 signal (2.49 ppm for proton and 39.5 ppm for carbon).

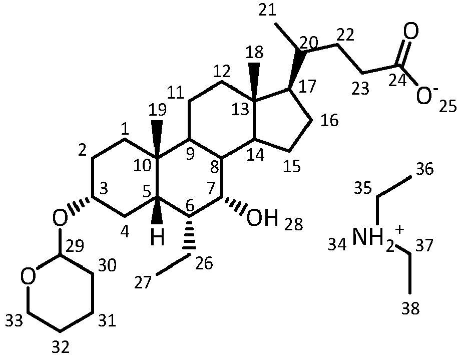

**Table 1**

| *Atom* | *δ (ppm)* | *No. protons* | *Multiplicity* | *δ (ppm)* | *Observations* |
|---|---|---|---|---|---|
| 1 | 1.72-0.89 | 2H | m | 35.35 | CH₂ |
| 2 | 1.60-1.27 | 2H | m | 28.46 | CH₂ |
| 3 | 3.26 | 1H | m | 76.35 | CH |
| 4 | 1.80-1.60 | 2H | m | 29.7 | CH₂ |
| 5 | 1.16 | 1H | m | 45.26 | CH |
| 6 | 1.43 | 1H | m | 41.26 | CH |
| 7 | 3.49 | 1H | m | 68.26 | CH |
| 8 | 1.29 | 1H | m | 39.62 | CH |
| 9 | 1.67 | 1H | m | 32.56 | CH |
| 10 | - | - | - | 35.31 | Cq |
| 11 | 1.9-1.15 | 2H | m | 20.36 | CH₂ |
| 12 | 1.89-1.10 | 2H | m | 39.29 | CH₂ |
| 13 | - | - | - | 42.87 | Cq |
| 14 | 1.40 | 1H | m | 50.04 | CH |
| 15 | 1.71-0.97 | 2H | m | 23.1 | CH₂ |
| 16 | 1.76-1.22 | 2H | m | 27.84 | CH₂ |
| 17 | 1.06 | 1H | m | 55.65 | CH |
| 18 | 0.59 | 3H | m | 11.70 | CH₃ |
| 19 | 0.83 | 3H | m | 18.25 | CH₃ |
| 20 | 1.33 | 1H | m | 35.04 | CH |
| 21 | 0.86 | 3H | m | 23.0 | CH₃ |
| 22 | 1.66-1.15 | 2H | m | 31.1 | CH₂ |
| 23 | 2.14-2.02 | 2H | m | 31.81 | CH₂ |
| 24 | - | - | - | 175.43 | Cq |
| 25 | - | - | - | - | O- |
| 26 | 1.43-1.22 | 2H | m | 22.1 | CH₂ |
| 27 | 0.81 | 3H | m | 11.7 | CH₃ |
| 28 | - | - | | - | OH |
| 29 | 4.65 | 1H | m | 96.2 | CH |
| 30 | 1.57-1.38 | 2H | m | 30.90 | CH₂ |
| 31 | 1.69-1.43 | 2H | m | 19.37 | CH₂ |
| 32 | 1.41 | 2H | m | 25.14 | CH₂ |
| 33 | 3.74-3.37 | 2H | m | 61.39 | CH₂ |
| 34 | 8.30 | NH₂⁺ | s | - | NH |
| 35 and 37 | 2.55 | 4H | q | 42.87 | CH₂ |
| 36 and 38 | 1.0 | 6H | t | 14.40 | CH₃ |

### Example 8. Obtaining of an amorphous form of obeticholic acid (compound of formula (II)) from the diethylamine salt of 3α-tetrahydropyranyloxy-6α-ethyl-7α-hydroxy-5β-cholanic acid (compound of formula (I))

46.2 g (79.9 mmol) of the diethylamine salt of 3α-tetrahydropyranyloxy-6α-ethyl-7α-hydroxy-5β-cholanic acid (I) were mixed with 462 mL of methanol. 90 mL (180 mmol, 2.25 molar eq.) of a 2N aqueous solution of HCl were added, maintaining the temperature between 20 and 25°C, and the mixture thus obtained was maintained under stirring at the temperature between 20 and 25°C for 8 hours.

Once this maintenance was finished, the pH of the reaction mixture was adjusted to an approximate value of 12 by adding 100 mL of a 30% aqueous solution of sodium hydroxide. The obtained mixture was heated at the temperature of about 50°C and was maintained at said temperature for 5 hours.

Once this maintenance was finished, the solvent was removed by distillation under reduced pressure and water was added to a total approximate volume of 1 L. The obtained mixture was filtered, and the obtained filtered solution was slowly added to a 12 N aqueous solution of HCl, maintaining the temperature between 20 and 25°C. The final pH resulting from the mixture was about 1.

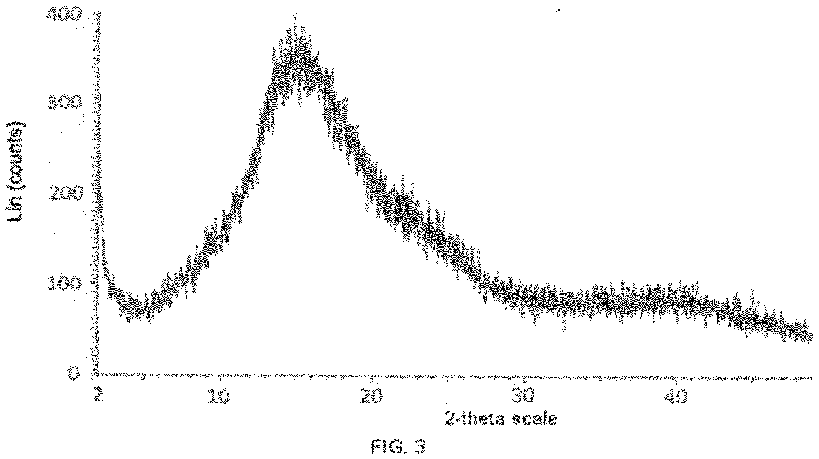

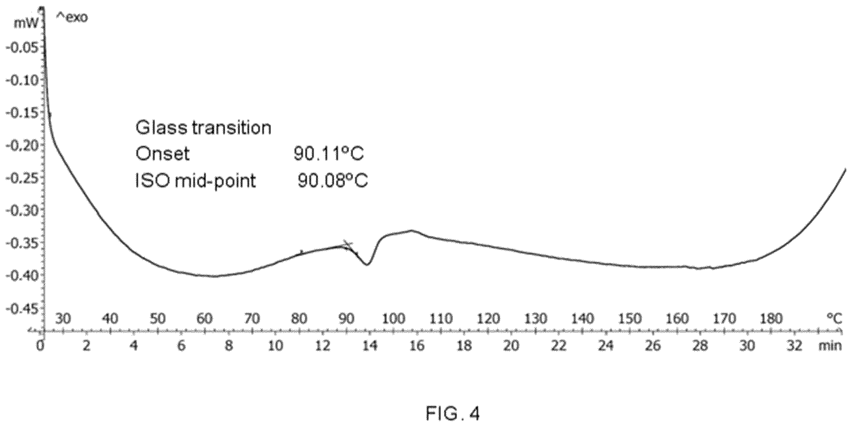

The obtained mixture was left stirring at the temperature between 20 and 25°C for 15 minutes once the addition was finished. The resulting solid was filtered and dried in an air oven at the temperature of 50°C. 28.64 g (85.2% yield) of a white solid corresponding to obeticholic acid (II) were thereby obtained. The purity of the product was analyzed by HPLC, obtaining 99.86%. Figure 3 shows the XRPD of the obtained product and Figure 4 shows its DSC.

### Example 9. Obtaining of different amine salts of 3α-tetrahydropyranyloxy-6α-ethyl-7α-hydroxy-5β-cholanic acid

3α-tetrahydropyranyloxy-6α-ethyl-7α-hydroxy-5β-cholanic acid (Ia) used in the experiments described below can be obtained by following the methodology described in Example 7 up to the point of the process where a solution of 3α-tetrahydropyranyloxy-6α-ethyl-7α-hydroxy-5β-cholanic acid (Ia) in ethyl acetate is obtained. n-Heptane is added to the resulting solution, and it is slowly cooled to the temperature of about 20°C, observing the presence of a white solid. The solid can be filtered so as to thereby isolate the desired product with a purity analyzed by HPLC of 95.48%.

Recrystallizing the product in 4 volumes of ethyl acetate allows obtaining a product with a purity analyzed by HPLC of 97.04% and a yield of 63.0%.

### Example 9.1. Obtaining of the diethylamine salt of 3α-tetrahydropyranyloxy-6α-ethyl-7α-hydroxy-5β-cholanic acid (compound of formula (I))

5 g of 3α-tetrahydropyranyloxy-6α-ethyl-7α-hydroxy-5β-cholanic acid (Ia) (9.9 mmol) and 1.2 mL of diethylamine (11.88 mmol) were mixed with 25 mL of ethyl acetate at the temperature between 20 and 25°C. The mixture was heated at reflux temperature and maintained at said temperature for 30 minutes.

The resulting solution was slowly cooled at the temperature of 20 and 25°C, and the obtained mixture was maintained for 16 hours at said temperature. The resulting solid was filtered and dried in an air oven at the temperature of 50°C. 4.82 g (84.2% yield) of a white solid corresponding to the diethylamine salt of 3α-tetrahydropyranyloxy-6α-ethyl-7α-hydroxy-5β-cholanic acid (I) were thereby obtained. The purity of the product was analyzed by HPLC, obtaining 98.98%.

The diethylamine salt (I) is characterized by having a differential scanning calorimetry (DSC) graph with an endothermic peak at an onset temperature *("T onset")* of about 202°C, as shown in Figure 2.

### Comparative Example 9.2. Obtaining of the trometamol salt of 3α-tetrahydropyranyloxy-6α-ethyl-7α-hydroxy-5β-cholanic acid

5 g of 3α-tetrahydropyranyloxy-6α-ethyl-7α-hydroxy-5β-cholanic acid (Ia) (9.9 mmol) and 1.2 g of trometamol (2-amino-2-(hydroxymethyl)-1,3-propanodiol) (9.9 mmol) were mixed with 25 mL of methanol at the temperature between 20-25°C. The mixture was heated at reflux temperature and maintained at said temperature for 30 minutes.

The solvent was vacuum distilled , and 25 mL of ethyl acetate were added to the resulting residue. The mixture was heated at reflux temperature and maintained at said temperature for 30 minutes. It was slowly cooled at the temperature of 20 and 25°C, and the obtained mixture was maintained for 16 hours at said temperature. The resulting solid was filtered and dried in an air oven at the temperature of 50°C. 3.8 g (61.3% yield) of a white solid corresponding to the trometamol salt of 3α-tetrahydropyranyloxy-6α-ethyl-7α-hydroxy-5β-cholanic acid were thereby obtained. The purity of the product was analyzed by HPLC, obtaining 96.95%.

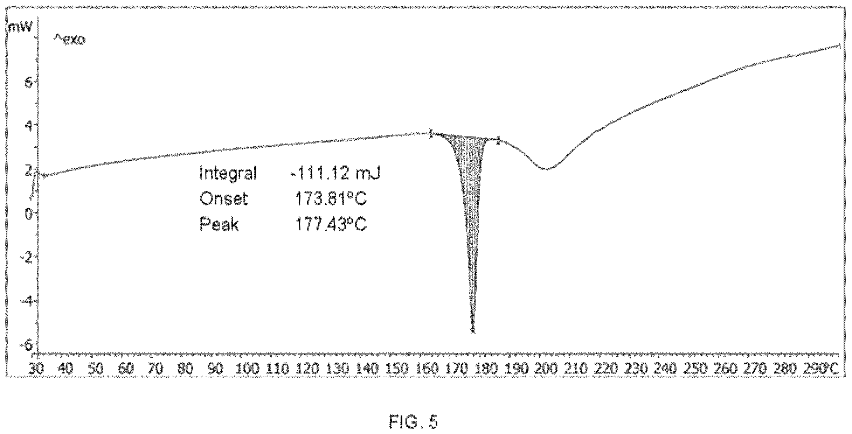

The trometamol salt is characterized by having a differential scanning calorimetry (DSC) graph with an endothermic peak at an onset temperature *("T onset")* of about 174°C, as shown in Figure 5.

### Comparative Example 9.3. Obtaining of the glycine salt of 3α-tetrahydropyranyloxy-6α-ethyl-7α-hydroxy-5β-cholanic acid

5 g of 3α-tetrahydropyranyloxy-6α-ethyl-7α-hydroxy-5β-cholanic acid (Ia) (9.9 mmol) and 0.743 g of glycine (9.9 mmol) were mixed with 25 mL of methanol and 25 mL of water at the temperature between 20 and 25°C. The mixture was heated at reflux temperature and maintained at said temperature for 30 minutes.

The resulting slightly cloudy solution was slowly cooled at the temperature of 20 and 25°C, and the obtained mixture was maintained for 16 hours at said temperature. The resulting solid was filtered and dried in an air oven at the temperature of 50°C. 4.4 g (76.7% yield) of a white solid corresponding to the glycine salt of 3α-tetrahydropyranyloxy-6α-ethyl-7α-hydroxy-5β-cholanic acid were thereby obtained. The purity of the product was analyzed by HPLC, obtaining 98.97%.

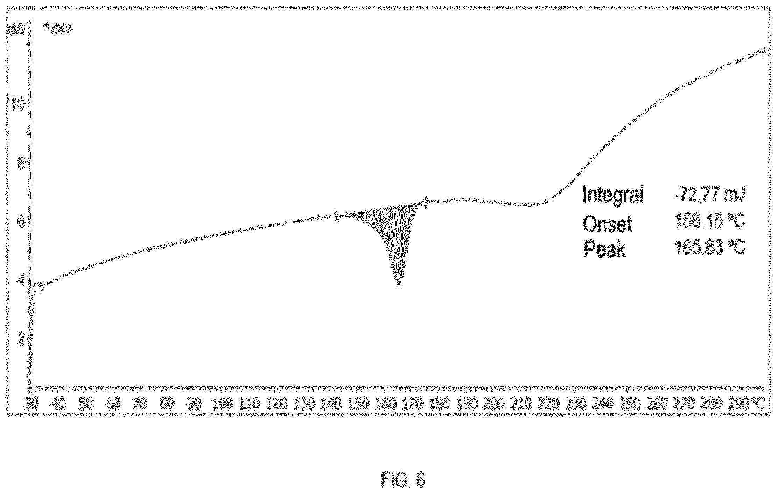

The glycine salt is characterized by having a differential scanning calorimetry (DSC) graph with an endothermic peak at an onset temperature *("T onset")* of about 158°C, as shown in Figure 6.

### Comparative Example 9.4. Obtaining of the taurine salt of 3α-tetrahydropyranyloxy-6α-ethyl-7α-hydroxy-5β-cholanic acid

5 g of 3α-tetrahydropyranyloxy-6α-ethyl-7α-hydroxy-5β-cholanic acid (Ia) (9.9 mmol) and 1.24 g of taurine (9.9 mmol) were mixed with 25 mL of methanol and 25 mL of water at the temperature between 20 and 25°C. The mixture was heated at reflux temperature and maintained at said temperature for 30 minutes.

The resulting slightly cloudy solution was slowly cooled at the temperature of 20 and 25°C, and the obtained mixture was maintained for 16 hours at said temperature. The resulting solid was filtered and dried in an air oven at the temperature of 50°C. 5.2 g (83.9% yield) of a white solid corresponding to the taurine salt of 3α-tetrahydropyranyloxy-6α-ethyl-7α-hydroxy-5β-cholanic acid were thereby obtained. The purity of the product was analyzed by HPLC, obtaining 97.54%.

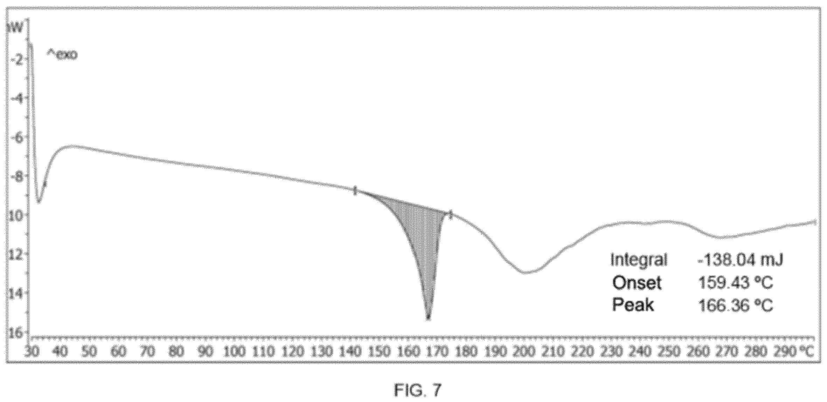

The taurine salt is characterized by having a differential scanning calorimetry (DSC) graph with an endothermic peak at an onset temperature *("T onset")* of about 159°C, as shown in Figure 7.

### Comparative Example 9.5. Obtaining of the triethylamine salt of 3α-tetrahydropyranyloxy-6α-ethyl-7α-hydroxy-5β-cholanic acid

5 g of 3α-tetrahydropyranyloxy-6α-ethyl-7α-hydroxy-5β-cholanic acid (Ia) (9.9 mmol) and 1.7 g of triethylamine (11.88 mmol) were mixed with 25 mL of acetone and 25 mL of water at the temperature between 20 and 25°C. The mixture was heated at reflux temperature and maintained at said temperature for 30 minutes.

The resulting slightly cloudy solution was slowly cooled at the temperature of 20 and 25°C, and the obtained mixture was maintained for 16 hours at said temperature. The resulting solid was filtered and dried in an air oven at the temperature of 50°C. 4.2 g (70.0% yield) of a white solid corresponding to the triethylamine salt of 3α-tetrahydropyranyloxy-6α-ethyl-7α-hydroxy-5β-cholanic acid were thereby obtained. The purity of the product was analyzed by HPLC, obtaining 99.36%.

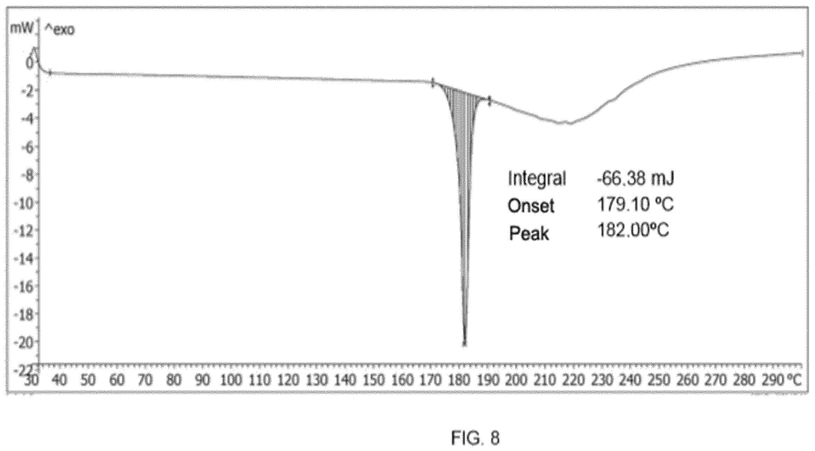

The triethylamine salt is characterized by having a differential scanning calorimetry (DSC) graph with an endothermic peak at an onset temperature *("T onset")* of about 179°C, as shown in Figure 8.

## Claims

1. A diethylamine salt of 3α-tetrahydropyranyloxy-6α-ethyl-7α-hydroxy-5β-cholanic acid of formula (I)

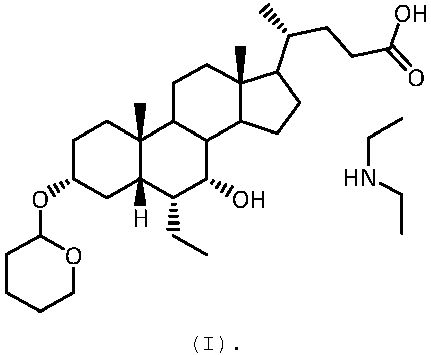

2. The diethylamine salt of formula (I) according to claim 1, **characterized in that** the diethylamine and 3α-tetrahydropyranyloxy-6o;-ethyl-7o;-hydroxy-5β-cholanic acid are at an approximate molar ratio of 1:1.

3. The diethylamine salt of formula (I) according to any one of the preceding claims, **characterized in that** it is in solid form.

4. The diethylamine salt of formula (I) according to any one of the preceding claims, **characterized in that** it has an X-ray powder diffraction pattern having peaks at 5.5, 7.8, 10.1, 11.1, 12.1, 12.8, 13.2, 14.2, 16.4, 16.7, 17.9, 20.3, 20.5, 22.1, and 23.3°2θ ± 0.2°2θ.

5. Use of the diethylamine salt of formula (I) as defined in any one of the preceding claims in a method for the preparation of obeticholic acid of formula (II)

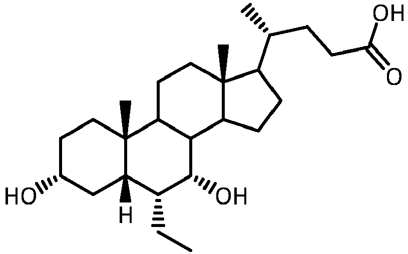

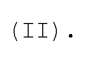

6. The use according to claim 5, wherein the method for the preparation of obeticholic acid of formula (II) comprises treating the diethylamine salt of formula (I) with an acid, preferably hydrochloric acid at a pH of 0 to 3 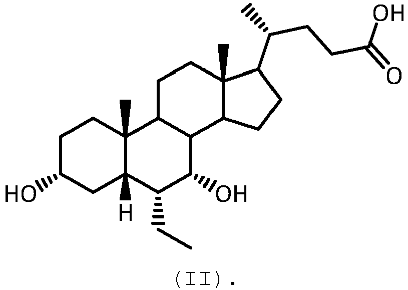 wherein the treatment is performed in the presence of a solvent preferably selected from the group consisting of C₁-C₄ alcohols, ketones, C₁-C₄ alkyl acetates, cyclic or linear ethers, acetonitrile, water, and mixtures thereof, preferably a mixture of methanol and water.

7. A method for the preparation of the diethylamine salt of formula (I) as defined in any one of claims 1 to 4, which method comprises treating 3α-tetrahydropyranyloxy-6α-ethyl-7o;-hydroxy-5β-cholanic acid (Ia) with diethylamine

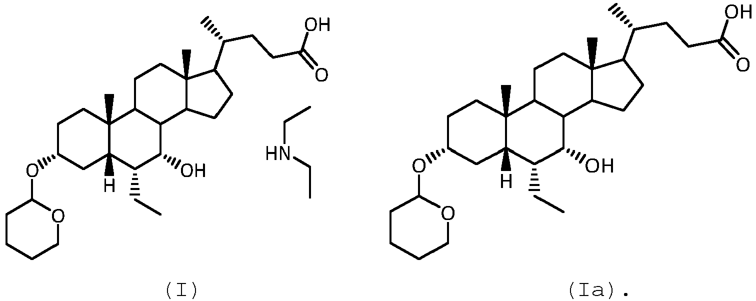

8. The method according to claim 7, wherein the treatment of 3α-tetrahydropyranyloxy-6α-ethyl-7α-hydroxy-5β-cholanic acid (Ia) with diethylamine is performed in the presence of a solvent selected from the group consisting of C₁-C₄ alkyl acetates, C₁-C₄ alcohols, ketones, water, and mixtures thereof, preferably ethyl acetate.

9. The method according to any one of claims 7 to 8, wherein 3α-tetrahydropyranyloxy-6α-ethyl-7α-hydroxy-5β-cholanic acid (Ia) is obtained by a method which comprises:
(a) treating a compound of formula (III) or a salt thereof, preferably the sodium salt of the compound of formula (III) with a reducing agent, preferably in an amount of comprised between 1 and 1.5 mol of reducing agent with respect to every mol of the compound of formula (III) or the salt thereof, to yield 3α-tetrahydropyranyloxy-6o;-ethyl-7o;-hydroxy-5β-cholanic acid (Ia) or a salt thereof 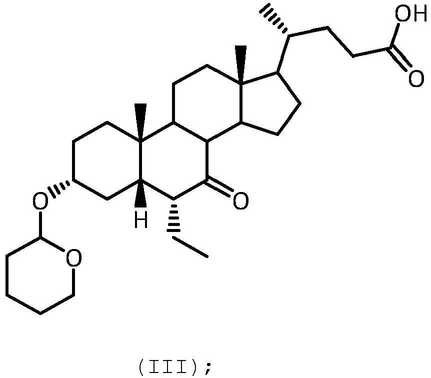 preferably in the presence of a solvent selected from the group consisting of C₁-C₃ alcohol, water, and mixtures thereof, preferably a mixture of methanol and water, preferably at a temperature between 20°C and 60°C and;
(b) optionally treating the salt of 3α-tetrahydropyranyloxy-6α-ethyl-7α-hydroxy-5β-cholanic acid (Ia) with an acid, preferably phosphoric acid, at a pH of 4 to 6 to yield 3α-tetrahydropyranyloxy-6α-ethyl-7α-hydroxy-5β-cholanic acid (Ia);
wherein the salt of the compound of formula (III) and the salt of 3α-tetrahydropyranyloxy-6α-ethyl-7α-hydroxy-5β-cholanic acid (Ia) are not diethylamine salts.

10. The method according to claim 9, wherein step (a) comprises treating a salt of a compound of formula (III) with sodium borohydride as the reducing agent to yield a salt of 3α-tetrahydropyranyloxy-6α-ethyl-7α-hydroxy-5β-cholanic acid (Ia), and wherein step (b) is performed.

11. The method according to any one of claims 9 to 10, wherein the compound of formula (III) or a salt thereof is obtained by a method which comprises:
(a) treating, preferably at a temperature between 35°C and 45°C, a compound of formula (IV) or a geometric isomer thereof with a base, preferably sodium hydroxide, preferably in an amount comprised between 1.5 and 2.5 mol, most preferably between 1.9 and 2.1 mol, of the base with respect to every mol of the compound of formula (IV) or a geometric isomer thereof and hydrogen, preferably at a hydrogen pressure between 4.5 and 5.5 bar, and in the presence of a catalyst, preferably selected from the group consisting of palladium on carbon, palladium on calcium carbonate, and platinum oxide, most preferably palladium on carbon, and preferably in the presence of a solvent selected from a C₁-C₃ alcohol, preferably methanol to yield a salt of the compound of formula (III) 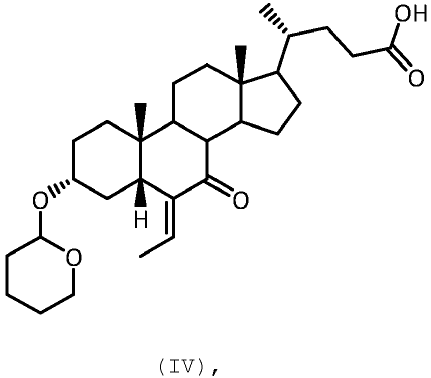 and
(b) optionally treating the salt of the compound of formula (III) with an acid at a pH of 4 to 6 to yield the compound of formula (III).

12. The method according to claim 11, wherein the compound of formula (III) is in the form of a salt and step (b) is not performed.

13. The method according to any one of claims 11 to 12, wherein the compound of formula (IV) or a geometric isomer thereof is obtained by treatment, preferably at a temperature between 15°C and 35°C, of a compound of formula (V) or a geometric isomer thereof with 3,4-dihydro-2H-pyran, preferably in an amount comprised between 1 and 2 mol of 3,4-dihydro-2H-pyran are used with respect to every mol of the compound of formula (V) or a geometric isomer thereof, in the presence of an acid, preferably camphorsulfonic acid, most preferably (1S)-(+)-10-camphorsulfonic acid, preferably in an amount comprised between 0.04 and 0.06 mol of the acid are used with respect to every mol of the compound of formula (V) or a geometric isomer thereof, preferably in the presence of a solvent selected from the group consisting of dichloromethane, tetrahydrofuran, and mixtures thereof, most preferably dichloromethane, wherein the solvent is preferably used in an amount comprised between 5 and 15 mL of solvent with respect to every gram of the compound of formula (V) or a geometric isomer thereof

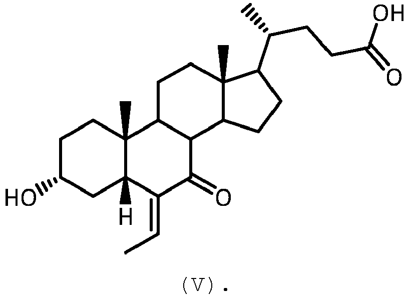

14. The method according to claim 13, wherein the compound of formula (V) or a geometric isomer thereof is obtained by treatment, preferably at a temperature between 40°C and 60°C of a compound of formula (VI) or a geometric isomer thereof in the presence of a base, preferably sodium hydroxide and a solvent selected from the group consisting of water, C₁-C₃ alcohol, and a mixture thereof, preferably a mixture of water and C₁-C₃ alcohol, most preferably a mixture of water and methanol

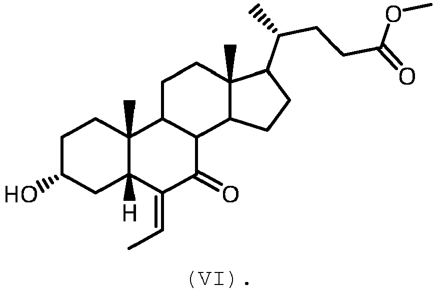

15. The method according to claim 14, wherein the compound of formula (VI) or a geometric isomer thereof is obtained by treatment of a compound of formula (VII) with acetaldehyde in the presence of boron trifluoride-diethyl ether

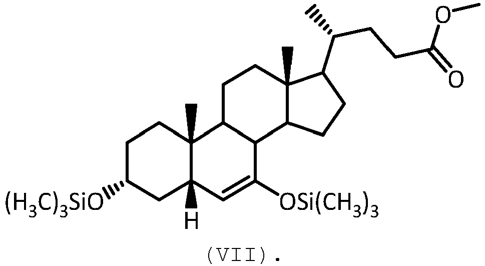

16. The method according to claim 15, wherein the compound of formula (VII) is obtained by treatment of a compound of formula (VIII) with chlorotrimethylsilane or trimethylsilyl trifluoromethanesulfonate in the presence of a base

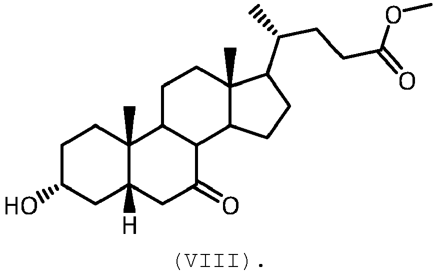

17. The method according to claim 16, wherein the compound of formula (VIII) is obtained by treatment of a compound of formula (IX) with methanol in the presence of an acid

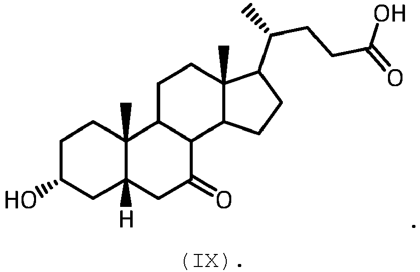

18. The method according to claim 17, wherein the compound of formula (IX) is obtained by treatment of a compound of formula (X) with sodium bromide and sodium hypochlorite

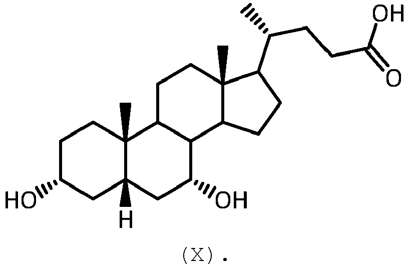

## Patentansprüche

1. Diethylamin-Salz von 3α-Tetrahydropyranyloxy-6α-ethyl-7α-hydroxy-5β-cholansäure der Formel (I)

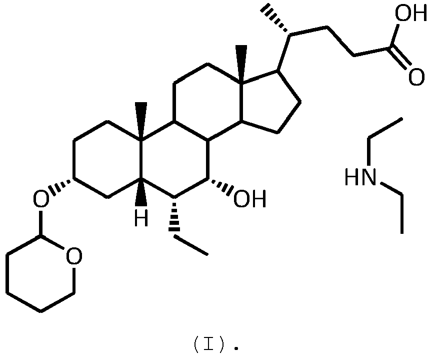

2. Diethylamin-Salz der Formel (I) nach Anspruch 1, **dadurch gekennzeichnet, dass** das Diethylamin und die 3α-Tetrahydropyranyloxy-6α-ethyl-7α-hydroxy-5β-cholansäure in einem ungefähren Molverhältnis von 1:1 vorliegen.

3. Diethylamin-Salz der Formel (I) nach einem der vorhergehenden Ansprüche, **dadurch gekennzeichnet, dass** es als Feststoff vorliegt.

4. Diethylamin-Salz der Formel (I) nach einem der vorhergehenden Ansprüche, **dadurch gekennzeichnet, dass** es ein Röntgenpulverbeugungsmuster mit Peaks bei 5.5, 7.8, 10.1, 11.1, 12.1, 12.8, 13.2, 14.2, 16.4, 16.7, 17.9, 20.3, 20.5, 22.1, and 23.3°2θ ± 0.2°2θ aufweist.

5. Verwendung des Diethylamin-Salzes der Formel (I), wie in einem der vorhergehenden Ansprüche definiert, in einem Verfahren zur Herstellung von Obeticholsäure der Formel (II)

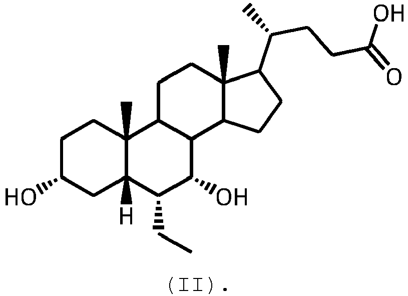

6. Verwendung nach Anspruch 5, wobei das Verfahren zur Herstellung von Obeticholsäure der Formel (II) 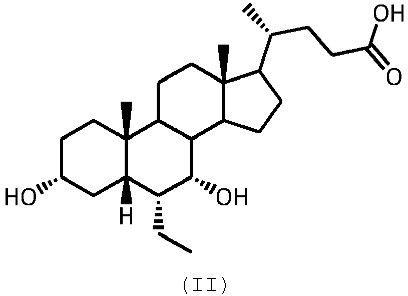 das Behandeln des Diethylamin-Salzes der Formel (I) mit einer Säure, vorzugsweise Salzsäure bei einem pH von 0 bis 3, umfasst;
wobei die Behandlung in Gegenwart eines Lösungsmittels durchgeführt wird, das vorzugsweise ausgewählt ist aus der Gruppe, bestehend aus C₁-C₄ Alkoholen, Ketonen, C₁-C₄ Alkylacetaten, zyklischen oder linearen Ethern, Acetonitril, Wasser, und Gemischen davon, vorzugsweise einem Gemisch aus Methanol und Wasser.

7. Verfahren zur Herstellung des Diethylamin-Salzes der Formel (I), wie in einem der Ansprüche 1 bis 4 definiert, wobei das Verfahren das Behandeln von 3α-Tetrahydropyranyloxy-6α-ethyl-7α-hydroxy-5β-cholansäure (Ia) mit Diethylamin 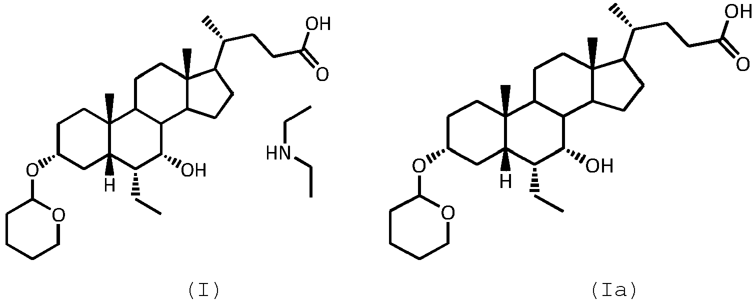 umfasst.

8. Verfahren nach Anspruch 7, wobei das Behandeln von 3α-Tetrahydropyranyloxy-6α-ethyl-7α-hydroxy-5β-cholansäure (Ia) mit Diethylamin in Gegenwart eines Lösungsmittels durchgeführt wird, das vorzugsweise ausgewählt ist aus der Gruppe, bestehend aus C₁-C₄ Alkylacetaten, C₁-C₄ Alkoholen, Ketonen, Wasser, und Gemischen davon, vorzugsweise Ethylacetat.

9. Verfahren nach einem der Ansprüche 7 bis 8, wobei 3α-Tetrahydropyranyloxy-6α-ethyl-7α-hydroxy-5β-cholansäure (Ia) durch ein Verfahren erhalten wird, welches umfasst:
(a) Behandeln einer Verbindung der Formel (III) 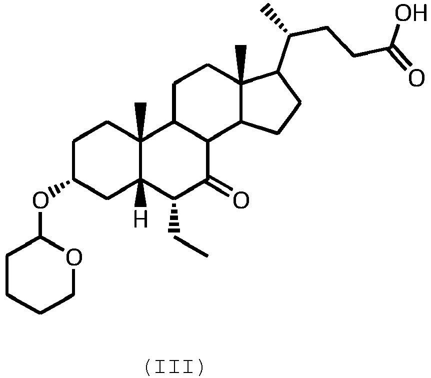 oder eines Salzes davon, vorzugsweise des Natriumsalzes der Verbindung der Formel (III), mit einem Reduktionsmittel, vorzugsweise in einer Menge, umfassend zwischen 1 und 1,5 Mol des Reduktionsmittels in Bezug auf jedes Mol der Verbindung der Formel (III) oder des Salzes davon, um 3α-Tetrahydropyranyloxy-6α-ethyl-7α-hydroxy-5β-cholansäure (Ia) oder ein Salz davon zu ergeben; 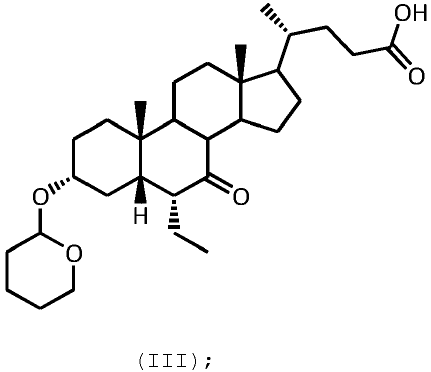 vorzugsweise in Gegenwart eines Lösungsmittels, das aus der Gruppe ausgewählt ist, bestehend aus C₁-C₃ Alkohol, Wasser, und Gemischen davon, vorzugsweise einem Gemisch aus Methanol und Wasser, vorzugsweise bei einer Temperatur zwischen 20°C und 60°C und;
(b) wahlweise Behandeln des Salzes von 3α-Tetrahydropyranyloxy-6α-ethyl-7α-hydroxy-5β-cholansäure (Ia) mit einer Säure, vorzugsweise Phosphorsäure, bei einem pH von 4 bis 6, um 3α-Tetrahydropyranyloxy-6α-ethyl-7α-hydroxy-5β-cholansäure (Ia) zu ergeben;
wobei das Salz der Verbindung der Formel (III) und das Salz von 3α-Tetrahydropyranyloxy-6α-ethyl-7α-hydroxy-5β-cholan-säure (Ia) keine Diethylamin-Salze sind.

10. Verfahren nach Anspruch 9, wobei Schritt (a) das Behandeln eines Salzes einer Verbindung der Formel (III) mit Natriumborhydrid als Reduktionsmittel umfasst, um ein Salz von 3α-Tetrahydropyranyloxy-6α-ethyl-7α-hydroxy-5β-cholan-säure (Ia) zu ergeben, und wobei Schritt (b) durchgeführt wird.

11. Verfahren nach einem der Ansprüche 9 bis 10, wobei die Verbindung der Formel (III) oder eines Salzes davon durch ein Verfahren erhalten wird, welches umfasst:
(a)Behandeln, vorzugsweise bei einer Temperatur zwischen 35°C und 45°C, einer Verbindung der Formel (IV) 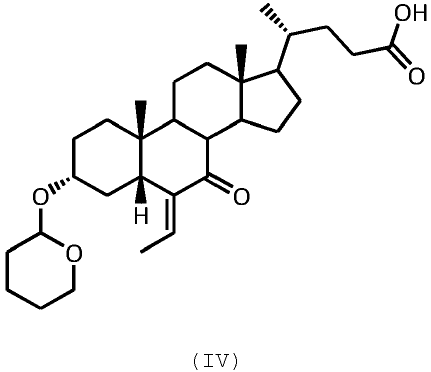 oder eines geometrischen Isomers davon, mit einer Base, vorzugsweise Natriumhydroxid, vorzugsweise in einer Menge, umfassend zwischen 1,5 und 2,5 Mol, am meisten bevorzugt zwischen 1,9 und 2,1 Mol, der Base in Bezug auf jedes Mol der Verbindung der Formel (IV) oder eines geometrischen Isomers davon, und Wasserstoff, vorzugsweise bei einem Wasserstoffdruck zwischen 4,5 und 5,5 bar, und in Gegenwart eines Katalysators, der vorzugsweise ausgewählt ist aus der Gruppe, bestehend aus Palladium auf Kohlenstoff, Palladium auf Calciumcarbonat, und Platinoxid, am meisten bevorzugt Palladium auf Kohlenstoff, und vorzugsweise in der Gegenwart eines Lösungsmittels, das ausgewählt ist aus einem C₁-C₃ Alkohol, vorzugsweise Methanol, um ein Salz der Verbindung der Formel (III) zu ergeben, und
(b)wahlweise Behandeln des Salzes der Verbindung der Formel (III) mit einer Säure bei einem pH von 4 bis 6, um die Verbindung der Formel (III) zu ergeben.

12. Verfahren nach Anspruch 11, wobei die Verbindung der Formel (III) in Gestalt eines Salzes vorliegt und Schritt (b) nicht durchgeführt wird.

13. Verfahren nach einem der Ansprüche 11 bis 12, wobei die Verbindung der Formel (IV) oder ein geometrisches Isomer davon erhalten wird durch Behandeln, vorzugsweise bei einer Temperatur zwischen 15°C und 35°C, einer Verbindung der Formel (V) 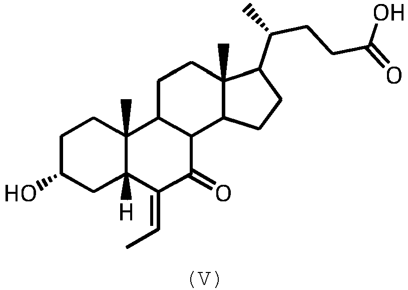 oder eines geometrischen Isomers davon mit 3,4-Dihydro-2H-pyran, vorzugsweise in einer Menge, umfassend zwischen 1 und 2 Mol von 3,4-Dihydro-2H-pyran in Bezug auf jedes Mol der Verbindung der Formel (V) oder eines geometrischen Isomers davon, in Gegenwart einer Säure, vorzugsweise Kampfersulfonsäure, am meisten bevorzugt (1S)-(+)-10-Kampfersulfonsäure, vorzugsweise in einer Menge, umfassend zwischen 0,04 und 0,06 Mol der Säure in Bezug auf jedes Mol der Verbindung der Formel (V) oder eines geometrischen Isomers davon, vorzugsweise in Gegenwart eines Lösungsmittels, das ausgewählt ist aus der Gruppe, bestehend aus Dichlormethan, Tetrahydrofuran, und Gemischen davon, am meisten bevorzugt Dichlormethan, wobei das Lösungsmittel vorzugsweise in einer Menge, umfassend zwischen 5 und 15 mL des Lösungsmittels in Bezug auf jedes Gramm der Verbindung der Formel (V) oder eines geometrischen Isomers davon verwendet wird.

14. Verfahren nach Anspruch 13, wobei die Verbindung der Formel (V) oder ein geometrisches Isomer davon erhalten wird durch Behandlung, vorzugsweise bei einer Temperatur zwischen 40°C und 60°C, einer Verbindung der Formel (VI) 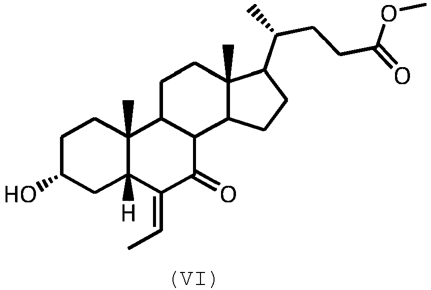 oder eines geometrischen Isomers davon in Gegenwart einer Base, vorzugsweise Natriumhydroxid, und eines Lösungsmittels, ausgewählt aus der Gruppe, bestehend aus Wasser, C₁-C₃ Alkohol und einem Gemisch davon, vorzugsweise eines Gemisches aus Wasser und C₁-C₃ Alkohol, am meisten bevorzugt eines Gemisches aus Wasser und Methanol.

15. Verfahren nach Anspruch 14, wobei die Verbindung der Formel (VI) oder ein geometrisches Isomer davon durch Behandlung einer Verbindung der Formel (VII) 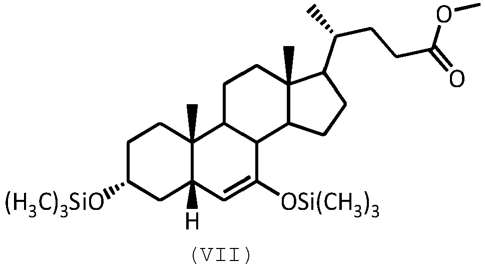 mit Acetaldehyd in Gegenwart von Bortrifluorid-diethylether erhalten wird.

16. Verfahren nach Anspruch 15, wobei die Verbindung der Formel (VII) durch Behandlung einer Verbindung der Formel (VIII) 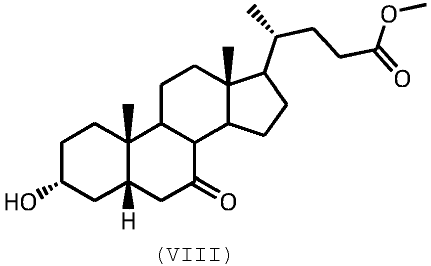 mit Chlortrimethylsilan oder Trimethylsilyltrifluormethansulfonat in Gegenwart einer Base erhalten wird.

17. Verfahren nach Anspruch 16, wobei die Verbindung der Formel (VIII) durch Behandlung einer Verbindung der Formel (IX) 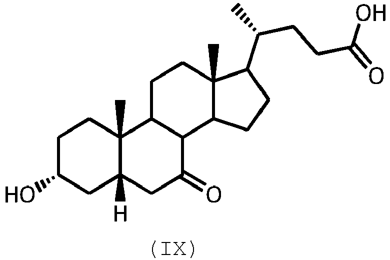 mit Methanol in Gegenwart einer Säure erhalten wird.

18. Verfahren nach Anspruch 17, wobei die Verbindung der Formel (IX) durch Behandlung einer Verbindung der Formel (X) 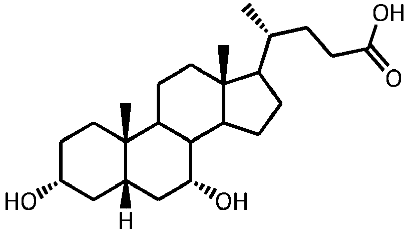 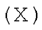 mit Natriumbromid und Natriumhypochlorit erhalten wird.

## Revendications

1. Sel de diéthylamine d'acide 3α-tétrahydropyranyloxy-6α-éthyl-7α-hydroxy-5β-cholanique de formule (I)

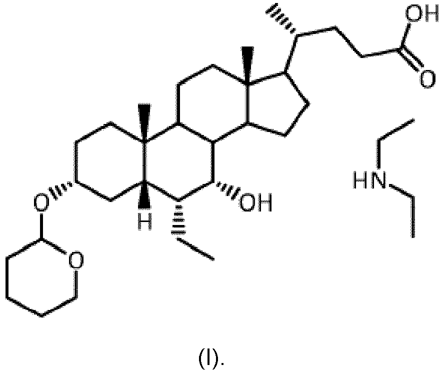

2. Sel de diéthylamine de formule (I) selon la revendication 1, **caractérisé en ce que** la diéthylamine et l'acide 3α-tétrahydropyranyloxy-6α-éthyl-7α-hydroxy-5β-cholanique sont à un rapport molaire d'environ 1:1.

3. Sel de diéthylamine de formule (I) selon l'une quelconque des revendications précédentes, **caractérisé en ce qu'**il se présente sous forme solide.

4. Sel de diéthylamine de formule (I) selon l'une quelconque des revendications précédentes, **caractérisé en ce qu'**il a un motif de diffraction de rayons X sur poudres présentant des pics à 5,5, 7,8, 10,1, 11,1, 12,1, 12,8, 13,2, 14,2, 16,4, 16,7, 17,9, 20,3, 20,5, 22,1 et 23,3°2θ ± 0,2°2θ.

5. Utilisation du sel de diéthylamine de formule (I) tel que défini dans l'une quelconque des revendications précédentes dans un procédé pour la préparation d'acide obéticholique de formule (II)

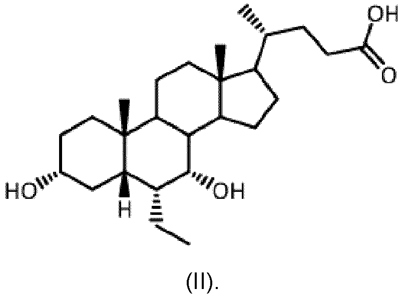

6. Utilisation selon la revendication 5, dans laquelle le procédé pour la préparation d'acide obéticholique de formule (II) comporte le traitement du sel de diéthylamine de formule (I) avec un acide, de préférence de l'acide chlorhydrique à un pH de 0 à 3 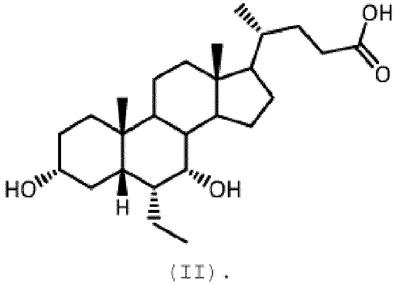 dans laquelle le traitement est réalisé en présence d'un solvant de préférence choisi parmi le groupe constitué d'alcools en C₁ à C₄, de cétones, d'acétates d'alkyle en C₁ à C₄, d'éthers cycliques ou linéaires, d'acétonitrile, d'eau et de mélanges de ceux-ci, de préférence un mélange de méthanol et d'eau.

7. Procédé pour la préparation du sel de diéthylamine de formule (I) tel que défini dans l'une quelconque des revendications 1 à 4, lequel procédé comporte le traitement de l'acide 3α-tétrahydropyranyloxy-6α-éthyl-7α-hydroxy-5β-cholanique (la) avec de la diéthylamine

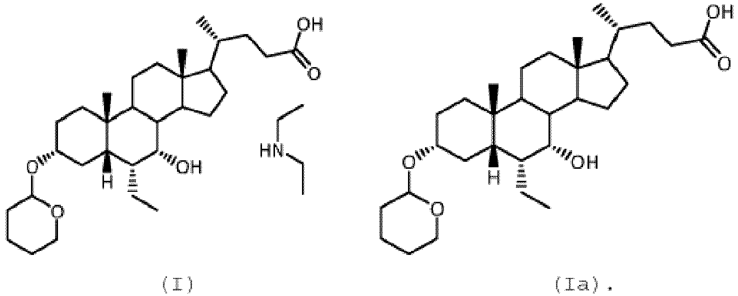

8. Procédé selon la revendication 7, dans lequel le traitement de l'acide 3α-tétrahydropyranyloxy-6α-éthyl-7α-hydroxy-5β-cholanique (la) avec de la diéthylamine est réalisé en présence d'un solvant choisi parmi le groupe constitué d'acétates d'alkyle en C₁ en C₄, d'alcools en C₁ à C₄, de cétones, d'eau et de mélanges de ceux-ci, de préférence de l'acétate d'éthyle.

9. Procédé selon l'une quelconque des revendications 7 à 8, dans lequel l'acide 3α-tétrahydropyranyloxy-6α-éthyl-7α-hydroxy-5β-cholanique (la) est obtenu par un procédé qui comporte les étapes consistant à :
(a) traiter un composé de formule (III) ou un sel de celui-ci, de préférence le sel de sodium du composé de formule (III) avec un agent réducteur, de préférence en une quantité comprise entre 1 et 1,5 mole d'agent réducteur par rapport à chaque mole du composé de formule (III) ou du sel de celui-ci, pour produire de l'acide 3α-tétrahydropyranyloxy-6α -éthyl-7α-hydroxy-5β-cholanique (la) ou un sel de celui-ci 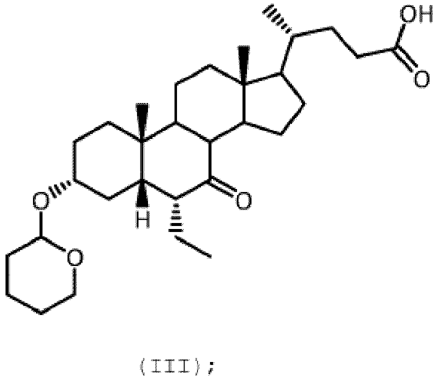 de préférence en présence d'un solvant choisi parmi le groupe constitué d'un alcool en C₁ à Ca, d'eau et de mélanges de ceux-ci, de préférence un mélange de méthanol et d'eau, de préférence à une température entre 20 °C et 60 °C et ;
(b) traiter facultativement le sel d'acide 3α-tétrahydropyranyloxy-6α-éthyl-7α-hydroxy-5β-cholanique (la) avec un acide, de préférence de l'acide phosphorique, à un pH de 4 à 6 pour produire de l'acide 3α-tétrahydropyranyloxy-6α-éthyl-7α-hydroxy-5β - cholanique (la) ;
dans lequel le sel du composé de formule (III) et le sel d'acide 3α-tétrahydropyranyloxy-6α-éthyl-7α-hydroxy-5β-cholanique (la) ne sont pas des sels de diéthylamine.

10. Procédé selon la revendication 9, dans lequel l'étape (a) comporte le traitement d'un sel d'un composé de formule (III) avec du borohydrure de sodium en tant qu'agent réducteur pour produire un sel d'acide 3α**-**tétrahydropyranyloxy-6α-éthyl-7α-hydroxy-5β-cholanique (la), et dans lequel l'étape (b) est mise en oeuvre.

11. Procédé selon l'une quelconque des revendications 9 à 10, dans lequel le composé de formule (III) ou un sel de celui-ci est obtenu par un procédé qui comporte les étapes consistant à :
(a) traiter, de préférence à une température entre 35 °C et 45 °C, un composé de formule (IV) ou un isomère géométrique de celui-ci avec une base, de préférence de l'hydroxyde de sodium, de manière préférée en une quantité comprise entre 1,5 et 2,5 moles, de manière la plus préférée entre 1,9 et 2,1 moles, de la base par rapport à chaque mole du composé de formule (IV) ou d'un isomère géométrique de celui-ci et d'hydrogène, de préférence à une pression d'hydrogène entre 4,5 et 5,5 bar, et en présence d'un catalyseur, de manière préférée choisi parmi le groupe constitué de palladium sur carbone, de palladium sur carbonate de calcium, et d'oxyde de platine, de manière la plus préférée de palladium sur carbone, et de préférence en présence d'un solvant choisi parmi un alcool en C₁ à Ca, de préférence du méthanol, pour produire un sel du composé de formule (III) 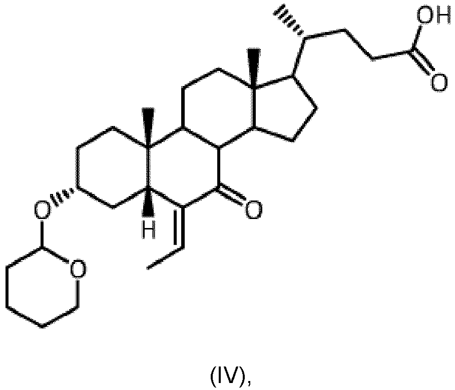 et
(b) traiter facultativement le sel du composé de formule (III) avec un acide à un pH de 4 à 6 pour produire le composé de formule (III).

12. Procédé selon la revendication 11, dans lequel le composé de formule (III) se présente sous la forme d'un sel et l'étape (b) n'est pas mise en oeuvre.

13. Procédé selon l'une quelconque des revendications 11 à 12, dans lequel le composé de formule (IV) ou un isomère géométrique de celui-ci est obtenu par traitement, de préférence à une température entre 15 °C et 35 °C, d'un composé de formule (V) ou d'un isomère géométrique de celui-ci avec du 3,4-dihydro-2H-pyrane, de préférence en utilisant une quantité comprise entre 1 et 2 moles de 3,4-dihydro-2H-pyrane par rapport à chaque mole du composé de formule (V) ou d'un isomère géométrique de celui-ci, en présence d'un acide, de manière préférée de l'acide camphorsulfonique, de manière la plus préférée de l'acide (1S)-(+)-10-camphorsulfonique, de préférence en utilisant une quantité comprise entre 0,04 et 0,06 mole de l'acide par rapport à chaque mole du composé de Formula (V) ou d'un isomère géométrique de celui-ci, de manière préférée en présence d'un solvant choisi parmi le groupe constitué de dichlorométhane, de tétrahydrofurane et de mélanges de ceux-ci, de manière la plus préférée de dichlorométhane, dans lequel le solvant est de préférence utilisé en une quantité comprise entre 5 et 15 ml de solvant par rapport à chaque gramme du composé de formule (V) ou d'un isomère géométrique de celui-ci

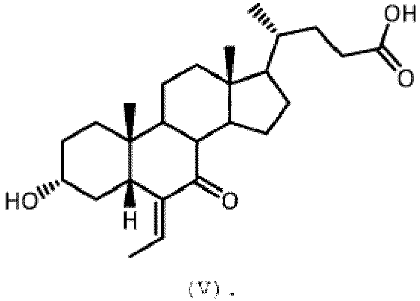

14. Procédé selon la revendication 13, dans lequel le composé de formule (V) ou un isomère géométrique de celui-ci est obtenu par traitement, de préférence à une température entre 40 °C et 60 °C d'un composé de formule (VI) ou d'un isomère géométrique de celui-ci en présence d'une base, de préférence de l'hydroxyde de sodium et d'un solvant choisi parmi le groupe constitué d'eau, d'un alcool en C₁ à C₃ et d'un mélange de ceux-ci, de manière préférée un mélange d'eau et d'alcool en C₁ à C₃, de manière la plus préférée un mélange d'eau et de méthanol

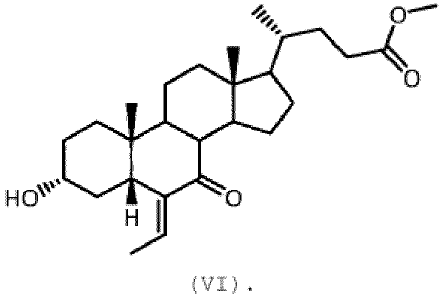

15. Procédé selon la revendication 14, dans lequel le composé de formule (VI) ou un isomère géométrique de celui-ci est obtenu par traitement d'un composé de formule (VII) avec un acétaldéhyde en présence d'un éther diéthylique de trifluorure de bore

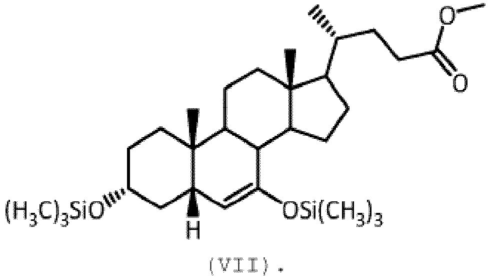

16. Procédé selon la revendication 15, dans lequel le composé de formule (VII) est obtenu par traitement d'un composé de formule (VIII) avec du chlorotriméthylsilane ou du triméthylsilyl trifluorométhanesulfonate en présence d'une base

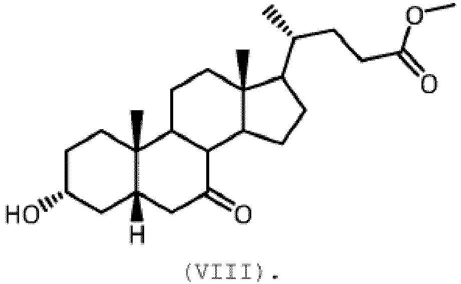

17. Procédé selon la revendication 16, dans lequel le composé de formule (VIII) est obtenu par traitement d'un composé de formule (IX) avec du méthanol en présence d'un acide

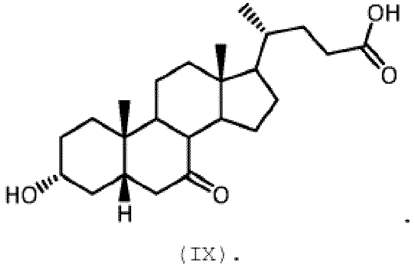

18. Procédé selon la revendication 17, dans lequel le composé de formule (IX) est obtenu par traitement d'un composé de formule (X) avec du bromure de sodium et de l'hypochlorite de sodium
